# EUROPEAN PATENT APPLICATION

(11) **EP 1 260 816 A1**
(43) Date of publication of application: **27.11.2002**
(21) Application number: 01906269.4
(22) Date of filing: 23.02.2001
(51) Int. Cl.: G01N 33/53, C12Q 1/68, C12N 15/12, G01N 33/15, A01K 67/027, A61K 39/395, A61P 37/08

(54) **METHOD OF EXAMINING ALLERGIC DISEASES**

(30) Priority: 02.03.2000 JP 2000061832
(71) Applicant: Genox Research, Inc., Ibaraki 300-2635 (JP); Japan as represented by General Director of Agency of National Center for Child Health and Development, Tokyo 157-8535 (JP)
(72) Inventor: NAGASU, Takeshi, c/o Eisai Co., Ltd., Tsukuba-shi, Ibaraki 300-2635 (JP); OSHIDA, Tadahiro, c/o Genox Research, Inc., Kawasaki-shi, Kanagawa 216-0001 (JP); OBAYASHI, Izumi, Yokohama-shi, Kanagawa 227-0038 (JP); MATSUI, Keiko, c/o Genox Research, Inc., Kawasaki-shi, Kanagawa 216-0001 (JP); SAITO, Hirohisa, Nat.Center Child Health and Dev., Tokyo 154-8509 (JP)
(74) Representative: Warcoin, Jacques
(86) International application number: JP0101372
(87) International publication number: WO01065259

(57) **Abstract**

The present inventors collected blood samples from multiple normal healthy subjects and allergic disease patients, and conducted differential display analysis to search for genes with different expression levels among them. As a result, the present inventors succeeded in isolating genes whose expression levels were increased in the patient group. The present inventors discovered that these genes can be utilized in testing for allergic disease, and screening for compounds that serve as candidate therapeutic agents for allergic disease.

SEQUENCE LISTING

<110> Genox Research, Inc.

<110> General Director of National Children's Hospital

<120> METHOD OF TESTING FOR ALLERGIC DISEASE

<130> 64856

<140>
<141>

<150> JP 2000-61832
<151> 2000-03-02

<150> PCT/JP 01/01372
<151> 2001-02-23

<160> 31

<170> PatentIn Ver. 2.0

<210> 1
<211> 1015
<212> DNA
<213> Homo sapiens

<220>
<221> CDS
<222> (1)..(615)

<400> 1
atg gcc gag ccg ctc cag cca gac ccc ggg gcg gcc gag gac gcg gcg     48
Met Ala Glu Pro Leu Gln Pro Asp Pro Gly Ala Ala Glu Asp Ala Ala
  1               5                  10                  15

gcc caa gct gtg gag acg ccg ggc tgg aag gcc ccg gag gac gcc ggc     96
Ala Gln Ala Val Glu Thr Pro Gly Trp Lys Ala Pro Glu Asp Ala Gly
             20                  25                  30

ccc cag ccc gga agt tat gag atc cga cac tat gga cca gcc aag tgg     144
Pro Gln Pro Gly Ser Tyr Glu Ile Arg His Tyr Gly Pro Ala Lys Trp
         35                  40                  45

gtc agc acg tcc gtg gag tct atg gac tgg gat tca gcc atc cag acg     192
Val Ser Thr Ser Val Glu Ser Met Asp Trp Asp Ser Ala Ile Gln Thr
     50                  55                  60

ggc ttt acg aaa ctg aac agc tac att caa ggc aaa aac gag aaa gag     240
Gly Phe Thr Lys Leu Asn Ser Tyr Ile Gln Gly Lys Asn Glu Lys Glu
 65                  70                  75                  80

atg aaa ata aag atg aca gct cca gtg aca agc tac gtg gag cct ggt     288
Met Lys Ile Lys Met Thr Ala Pro Val Thr Ser Tyr Val Glu Pro Gly
                 85                  90                  95

tca ggt cct ttt agt gag tct acc att acc att tcc ctg tat att ccc     336
Ser Gly Pro Phe Ser Glu Ser Thr Ile Thr Ile Ser Leu Tyr Ile Pro
            100                 105                 110

tct gaa cag caa ttt gat cca ccc agg cct tta gag tca gat gtc ttc    384
Ser Glu Gln Gln Phe Asp Pro Pro Arg Pro Leu Glu Ser Asp Val Phe
        115                 120                 125

att gaa gat aga gcc gaa atg act gtg ttt gta cgg tct ttc gat gga    432
Ile Glu Asp Arg Ala Glu Met Thr Val Phe Val Arg Ser Phe Asp Gly
    130                 135                 140

ttt tct agt gcc caa aag aat caa gaa caa ctt ttg aca tta gca agc    480
Phe Ser Ser Ala Gln Lys Asn Gln Glu Gln Leu Leu Thr Leu Ala Ser
145                 150                 155                 160

att tta agg gaa gat gga aaa gtt ttc gat gag aag gtt tac tac act    528
Ile Leu Arg Glu Asp Gly Lys Val Phe Asp Glu Lys Val Tyr Tyr Thr
                165                 170                 175

gca ggc tac aac agt cct gtc aaa ttg ctt aat aga aat aat gaa gtg    576
Ala Gly Tyr Asn Ser Pro Val Lys Leu Leu Asn Arg Asn Asn Glu Val
            180                 185                 190

tgg ttg att caa aaa aat gaa ccc acc aaa gaa aac gaa tgagaaaaat    625
Trp Leu Ile Gln Lys Asn Glu Pro Thr Lys Glu Asn Glu
        195                 200                 205

gaaaggaagt tctgctgtca gaggcaaaac atctgtttat catagacatc aacatgacct 685

ataagtaaag tgcgtgtcta gtgtcttcta ttgagagtac tactattaat taagcttatt 745

tccaatgtgc ctttttaatg cttgaagttt tatctacata cacaggtaac agaggacagt 805

agtctgtaaa catataaatc ggtcataact atcgtggtct ttatttctgt gaggatctag 865

ggaaatttca tgtcacttcc ctccttcact gcatcacaat catattccct tttttttttc 925

ttggatttgt gtcagttgga tgatatcccc tccagatagt atcaataaaa tgttaaaatt 985

caggaaaaaa aaaaaaaaaa aaaaaaaaaa                                  1015

<210> 2
<211> 205
<212> PRT
<213> Homo sapiens

<400> 2
Met Ala Glu Pro Leu Gln Pro Asp Pro Gly Ala Ala Glu Asp Ala Ala
1               5                   10                  15

Ala Gln Ala Val Glu Thr Pro Gly Trp Lys Ala Pro Glu Asp Ala Gly
            20                  25                  30

Pro Gln Pro Gly Ser Tyr Glu Ile Arg His Tyr Gly Pro Ala Lys Trp
        35                  40                  45

Val Ser Thr Ser Val Glu Ser Met Asp Trp Asp Ser Ala Ile Gln Thr
    50                  55                  60

Gly Phe Thr Lys Leu Asn Ser Tyr Ile Gln Gly Lys Asn Glu Lys Glu
65                  70                  75                  80

Met Lys Ile Lys Met Thr Ala Pro Val Thr Ser Tyr Val Glu Pro Gly

85                  90                  95

Ser Gly Pro Phe Ser Glu Ser Thr Ile Thr Ile Ser Leu Tyr Ile Pro
            100                 105                 110

Ser Glu Gln Gln Phe Asp Pro Pro Arg Pro Leu Glu Ser Asp Val Phe
        115                 120                 125

Ile Glu Asp Arg Ala Glu Met Thr Val Phe Val Arg Ser Phe Asp Gly
    130                 135                 140

Phe Ser Ser Ala Gln Lys Asn Gln Glu Gln Leu Leu Thr Leu Ala Ser
145                 150                 155                 160

Ile Leu Arg Glu Asp Gly Lys Val Phe Asp Glu Lys Val Tyr Tyr Thr
                165                 170                 175

Ala Gly Tyr Asn Ser Pro Val Lys Leu Leu Asn Arg Asn Asn Glu Val
            180                 185                 190

Trp Leu Ile Gln Lys Asn Glu Pro Thr Lys Glu Asn Glu
        195                 200                 205


<210> 3
<211> 1867
<212> DNA
<213> Homo sapiens

<220>
<221> CDS
<222> (195)..(992)

<400> 3
ggcacgaggg gagctgggag gctgcgagat ccctaccgca gtagccgcct ctgccgccgc 60

ggagcttccc gaacctcttc agccgcccgg agccgctccc ggagcccggc cgtagaggct 120

gcaatcgcag ccgggagccc gcagcccgcg ccccgagccc gccgccgccc ttcgagggcg 180

ccccaggccg cgcc atg gtg aag gtg acg ttc aac tcc gct ctg gcc cag   230
                Met Val Lys Val Thr Phe Asn Ser Ala Leu Ala Gln
                1               5                   10

aag gag gcc aag aag gac gag ccc aag agc ggc gag gag gcg ctc atc   278
Lys Glu Ala Lys Lys Asp Glu Pro Lys Ser Gly Glu Glu Ala Leu Ile
        15                  20                  25

atc ccc ccc gac gcc gtc gcg gtg gac tgc aag gac cca gat gat gtg   326
Ile Pro Pro Asp Ala Val Ala Val Asp Cys Lys Asp Pro Asp Asp Val
    30                  35                  40

gta cca gtt ggc caa aga aga gcc tgg tgt tgg tgc atg tgc ttt gga   374
Val Pro Val Gly Gln Arg Arg Ala Trp Cys Trp Cys Met Cys Phe Gly
45                  50                  55                  60

cta gca ttt atg ctt gca ggt gtt att cta gga gga gca tac ttg tac   422
Leu Ala Phe Met Leu Ala Gly Val Ile Leu Gly Gly Ala Tyr Leu Tyr
                65                  70                  75

aaa tat ttt gca ctt caa cca gat gac gtg tac tac tgt gga ata aag   470

Lys Tyr Phe Ala Leu Gln Pro Asp Asp Val Tyr Tyr Cys Gly Ile Lys
            80                  85                  90

tac atc aaa gat gat gtc atc tta aat gag ccc tct gca gat gcc cca     518
Tyr Ile Lys Asp Asp Val Ile Leu Asn Glu Pro Ser Ala Asp Ala Pro
        95                  100                 105

gct gct ctc tac cag aca att gaa gaa aat att aaa atc ttt gaa gaa     566
Ala Ala Leu Tyr Gln Thr Ile Glu Glu Asn Ile Lys Ile Phe Glu Glu
    110                 115                 120

gaa gaa gtt gaa ttt atc agt gtg cct gtc cca gag ttt gca gat agt     614
Glu Glu Val Glu Phe Ile Ser Val Pro Val Pro Glu Phe Ala Asp Ser
125                 130                 135                 140

gat cct gcc aac att gtt cat gac ttt aac aag aaa ctt aca gcc tat     662
Asp Pro Ala Asn Ile Val His Asp Phe Asn Lys Lys Leu Thr Ala Tyr
                145                 150                 155

tta gat ctt aac ctg gat aag tgc tat gtg atc cct ctg aac act tcc     710
Leu Asp Leu Asn Leu Asp Lys Cys Tyr Val Ile Pro Leu Asn Thr Ser
            160                 165                 170

att gtt atg cca ccc aga aac cta ctg gag tta ctt att aac atc aag     758
Ile Val Met Pro Pro Arg Asn Leu Leu Glu Leu Leu Ile Asn Ile Lys
        175                 180                 185

gct gga acc tat ttg cct cag tcc tat ctg att cat gag cac atg gtt     806
Ala Gly Thr Tyr Leu Pro Gln Ser Tyr Leu Ile His Glu His Met Val
    190                 195                 200

att act gat cgc att gaa aac att gat cac ctg ggt ttc ttt att tat     854
Ile Thr Asp Arg Ile Glu Asn Ile Asp His Leu Gly Phe Phe Ile Tyr
205                 210                 215                 220

cga ctg tgt cat gac aag gaa act tac aaa ctg caa cgc aga gaa act     902
Arg Leu Cys His Asp Lys Glu Thr Tyr Lys Leu Gln Arg Arg Glu Thr
                225                 230                 235

att aaa ggt att cag aaa cgt gaa gcc agc aat tgt ttc gca att cgg     950
Ile Lys Gly Ile Gln Lys Arg Glu Ala Ser Asn Cys Phe Ala Ile Arg
            240                 245                 250

cat ttt gaa aac aaa ttt gcc gtg gaa act tta att tgt tct             992
His Phe Glu Asn Lys Phe Ala Val Glu Thr Leu Ile Cys Ser
        255                 260                 265

tgaacagtca agaaaaacat tattgaggaa aattaatatc acagcataac cccacccttt 1052

acattttgtg cagtgattat tttttaaagt cttctttcat gtaagtagca aacagggctt 1112

tactatcttt tcatctcatt aattcaatta aaaccattac cttaaaattt ttttctttcg 1172

aagtgtggtg tcttttatat ttgaattagt aactgtatga agtcatagat aatagtacat 1232

gtcaccttag gtagtaggaa gaattacaat ttctttaaat catttatctg gatttttatg 1292

ttttattagc attttcaaga agacggatta tctagagaat aatcatatat atgcatacgt 1352

aaaaatggac cacagtgact tatttgtagt tgttagttgc cctgctacct agtttgttag 1412

tgcatttgag cacacatttt aattttcctc taattaaaat gtgcagtatt ttcagtgtca 1472

aatatattta actatttaga gaatgatttc cacctttatg ttttaatatc ctaggcatct 1532

gctgtaataa tattttagaa aatgtttgga atttaagaaa taacttgtgt tactaatttg 1592

tataacccat atctgtgcaa tggaatataa atatcacaaa gttgtttaac tagactgcgt 1652

gttgtttttc ccgtataata aaaccaaaga atagtttggt tcttcaaatc ttaagagaat 1712

ccacataaaa gaagaaacta ttttttaaaa attcacttct atatatacaa tgagtaaaat 1772

cacagatttt ttctttaaat aaaaataagt cattttaata actaaaccag attctttgtg 1832

gatactatta aagtaacatt taagcctcaa ccttg 1867

<210> 4
<211> 266
<212> PRT
<213> Homo sapiens

<400> 4
Met Val Lys Val Thr Phe Asn Ser Ala Leu Ala Gln Lys Glu Ala Lys
1               5                   10                  15

Lys Asp Glu Pro Lys Ser Gly Glu Glu Ala Leu Ile Ile Pro Pro Asp
            20                  25                  30

Ala Val Ala Val Asp Cys Lys Asp Pro Asp Asp Val Val Pro Val Gly
        35                  40                  45

Gln Arg Arg Ala Trp Cys Trp Cys Met Cys Phe Gly Leu Ala Phe Met
    50                  55                  60

Leu Ala Gly Val Ile Leu Gly Gly Ala Tyr Leu Tyr Lys Tyr Phe Ala
65                  70                  75                  80

Leu Gln Pro Asp Asp Val Tyr Tyr Cys Gly Ile Lys Tyr Ile Lys Asp
                85                  90                  95

Asp Val Ile Leu Asn Glu Pro Ser Ala Asp Ala Pro Ala Ala Leu Tyr
            100                 105                 110

Gln Thr Ile Glu Glu Asn Ile Lys Ile Phe Glu Glu Glu Glu Val Glu
        115                 120                 125

Phe Ile Ser Val Pro Val Pro Glu Phe Ala Asp Ser Asp Pro Ala Asn
    130                 135                 140

Ile Val His Asp Phe Asn Lys Lys Leu Thr Ala Tyr Leu Asp Leu Asn
145                 150                 155                 160

Leu Asp Lys Cys Tyr Val Ile Pro Leu Asn Thr Ser Ile Val Met Pro
                165                 170                 175

Pro Arg Asn Leu Leu Glu Leu Leu Ile Asn Ile Lys Ala Gly Thr Tyr
            180                 185                 190

Leu Pro Gln Ser Tyr Leu Ile His Glu His Met Val Ile Thr Asp Arg
        195                 200                 205

Ile Glu Asn Ile Asp His Leu Gly Phe Phe Ile Tyr Arg Leu Cys His
    210                 215                 220

Asp Lys Glu Thr Tyr Lys Leu Gln Arg Arg Glu Thr Ile Lys Gly Ile
225                 230                 235                 240

Gln Lys Arg Glu Ala Ser Asn Cys Phe Ala Ile Arg His Phe Glu Asn
                245                 250                 255

Lys Phe Ala Val Glu Thr Leu Ile Cys Ser
            260                 265


<210> 5
<211> 274
<212> DNA
<213> Homo sapiens

<400> 5
gtggaacgtc tgtaaatayg tttttataat tccatgtata gttggtgtac actcaaaacc 60

tgtccccggc agccagtgct ctctgtatag ggccataatg gaattctgaa gaaatcttgg 120

ggagggaagg ggagttggaa caaatgtctg ttccctggag gccagtccag tgctcagacc 180

tttagactca ttgtaagttg ccactgccaa catgagacca aagtgtgtga ctagtcaatg 240

aagtgcgaca gattaaagac tgatgctaaa cctc                              274


<210> 6
<211> 224
<212> DNA
<213> Homo sapiens

<400> 6
agttaaaaag tattttgcat tatttgcagt aagatgtctc tagcactgct caaagggcaa 60

attttaaaac ttgattggac gtttgcagct cactacagaa aggatgagag aagaagagaa 120

tgaaggaata gttcaactac gaatactaag aacccaggaa gctatcatac aaataatgaa 180

aatgagaaag aaaattagta atgctcagct gcagactgaa ttag                  224


<210> 7
<211> 17
<212> DNA
<213> Artificial Sequence

<220>
<223> Description of Artificial Sequence:Artificially
      Synthesized Primer Sequence

<400> 7
gttttttttt tttttta                                                 17


<210> 8
<211> 17

<212> DNA
<213> Artificial Sequence

<220>
<223> Description of Artificial Sequence:Artificially
      Synthesized Primer Sequence

<400> 8
gttttttttt ttttttc                                                    17


<210> 9
<211> 17
<212> DNA
<213> Artificial Sequence

<220>
<223> Description of Artificial Sequence:Artificially
      Synthesized Primer Sequence

<400> 9
gttttttttt ttttttg                                                    17


<210> 10
<211> 10
<212> DNA
<213> Artificial Sequence

<220>
<223> Description of Artificial Sequence:Artificially
      Synthesized Primer Sequence

<400> 10
ggctttcgat                                                            10


<210> 11
<211> 10
<212> DNA
<213> Artificial Sequence

<220>
<223> Description of Artificial Sequence:Artificially
      Synthesized Primer Sequence

<400> 11
caggagtact                                                            10


<210> 12
<211> 10
<212> DNA
<213> Artificial Sequence

<220>
<223> Description of Artificial Sequence:Artificially
      Synthesized Primer Sequence

<400> 12
tgacctgagt                                                            10

<210> 13
<211> 10
<212> DNA
<213> Artificial Sequence

<220>
<223> Description of Artificial Sequence:Artificially
      Synthesized Primer Sequence

<400> 13
tagtgaagcc                                                            10


<210> 14
<211> 24
<212> DNA
<213> Artificial Sequence

<220>
<223> Description of Artificial Sequence:Artificially
      Synthesized Primer Sequence

<400> 14
tcgaaagacc gtacaaacac agtc                                            24


<210> 15
<211> 21
<212> DNA
<213> Artificial Sequence

<220>
<223> Description of Artificial Sequence:Artificially
      Synthesized Primer Sequence

<400> 15
gatccaccca ggcctttaga g                                               21


<210> 16
<211> 29
<212> DNA
<213> Artificial Sequence

<220>
<223> Description of Artificial Sequence:Artificially
      Synthesized Probe Sequence

<400> 16
ttcggctcta tcttcaatga agacatctg                                       29


<210> 17
<211> 21
<212> DNA
<213> Artificial Sequence

<220>
<223> Description of Artificial Sequence:Artificially

Synthesized Primer Sequence

<400> 17
gcggctgtac caagaagatg a                                                21


<210> 18
<211> 18
<212> DNA
<213> Artificial Sequence

<220>
<223> Description of Artificial Sequence:Artificially
      Synthesized Primer Sequence

<400> 18
cgggccaaag tggacaag                                                    18


<210> 19
<211> 22
<212> DNA
<213> Artificial Sequence

<220>
<223> Description of Artificial Sequence:Artificially
      Synthesized Probe Sequence

<400> 19
tcccacaagc ggctaaaccc cg                                               22


<210> 20
<211> 23
<212> DNA
<213> Artificial Sequence

<220>
<223> Description of Artificial Sequence:Artificially
      Synthesized Primer Sequence

<400> 20
atgcagtctg tctttgggat gat                                              23


<210> 21
<211> 25
<212> DNA
<213> Artificial Sequence

<220>
<223> Description of Artificial Sequence:Artificially
      Synthesized Primer Sequence

<400> 21
cctgtgcctg cttaataaac tcaag                                            25


<210> 22
<211> 27
<212> DNA

<213> Artificial Sequence

<220>
<223> Description of Artificial Sequence:Artificially
      Synthesized Probe Sequence

<400> 22
aggcccagca aaaattcatc aggcttt                                          27


<210> 23
<211> 21
<212> DNA
<213> Artificial Sequence

<220>
<223> Description of Artificial Sequence:Artificially
      Synthesized Primer Sequence

<400> 23
cgtcatctgg ttgaagtgca a                                                21


<210> 24
<211> 21
<212> DNA
<213> Artificial Sequence

<220>
<223> Description of Artificial Sequence:Artificially
      Synthesized Primer Sequence

<400> 24
tgcatgtgct ttggactagc a                                                21


<210> 25
<211> 29
<212> DNA
<213> Artificial Sequence

<220>
<223> Description of Artificial Sequence:Artificially
      Synthesized Probe Sequence

<400> 25
tgctcctcct agaataacac ctgcaagca                                        29


<210> 26
<211> 1666
<212> DNA
<213> Homo sapiens

<400> 26
aggtgacgtt caactccgct ctggcccaga aggaggccaa gaaggacgag cccaagagcg 60

gcgaggaggc gctcatcatc ccccccgacg ccgtcgcggt ggactgcaag gacccagatg 120

atgtggtacc agttggccaa agaagagcct ggtgttggtg catgtgcttt ggactagcat 180

ttatgcttgc aggtgttatt ctaggaggag catacttgta caaatatttt gcacttcaac 240

cagatgacgt gtactactgt ggaataaagt acatcaaaga tgatgtcatc ttaaatgagc 300

cctctgcaga tgccccagct gctctctacc agacaattga agaaaatatt aaaatctttg 360

aagaagaaga agttgaattt atcagtgtgc ctgtcccaga gtttgcagat agtgatcctg 420

ccaacattgt tcatgacttt aacaagaaac ttacagccta tttagatctt aacctggata 480

agtgctatgt gatccctctg aacacttcca ttgttatgcc acccagaaac ctactggagt 540

tacttattaa catcaaggct ggaacctatt tgcctcagtc ctatctgatt catgagcaca 600

tggttattac tgatcgcatt gaaaacattg atcacctggg tttctttatt tatcgactgt 660

gtcatgacaa ggaaacttac aaactgcaac gcagagaaac tattaaaggt attcagaaac 720

gtgaagccag caattgtttc gcaattcggc attttgaaaa caaatttgcc gtggaaactt 780

taatttgttc ttgaacagtc aagaaaaaca ttattgagga aaattaatat cacagcataa 840

ccccaccctt tacattttgt gcagtgatta ttttttaaag tcttctttca tgtaagtagc 900

aaacagggct ttactatctt ttcatctcat taattcaatt aaaaccatta ccttaaaatt 960

tttttctttc gaagtgtggt gtcttttata tttgaattag taactgtatg aagtcataga 1020

taatagtaca tgtcacctta ggtagtagga agaattacaa tttctttaaa tcatttatct 1080

ggatttttat gttttattag cattttcaag aagacggatt atctagagaa taatcatata 1140

tatgcatacg taaaaatgga ccacagtgac ttatttgtag ttgttagttg ccctgctacc 1200

tagtttgtta gtgcatttga gcacacattt taattttcct ctaattaaaa tgtgcagtat 1260

tttcagtgtc aaatatattt aactatttag agaatgattt ccacctttat gttttaatat 1320

cctaggcatc tgctgtaata atattttaga aaatgtttgg aatttaagaa ataacttgtg 1380

ttactaattt gtataaccca tatctgtgca atggaatata aatatcacaa agttgtttaa 1440

ctagactgcg tgttgttttt cccgtataat aaaaccaaag aatagtttgg ttcttcaaat 1500

cttaagagaa tccacataaa agaagaaact attttttaaa aattcacttc tatatataca 1560

atgagtaaaa tcacagattt tttctttaaa taaaaataag tcattttaat aactaaacca 1620

gattctttgt ggatactatt aaagtaacat ttaagcctca accttg 1666

<210> 27
<211> 23
<212> DNA
<213> Artificial Sequence

<220>
<223> Description of Artificial Sequence:Artificially
      Synthesized Primer Sequence

<400> 27

gccaagaagg acgagcccaa gag                                          23

<210> 28
<211> 23
<212> DNA
<213> Artificial Sequence

<220>
<223> Description of Artificial Sequence:Artificially
      Synthesized Primer Sequence

<400> 28
aaagggtggg gttatgctgt gat                                          23

<210> 29
<211> 1647
<212> DNA
<213> Homo sapiens

<400> 29
ggcacgaggg gagctgggag gctgcgagat ccctaccgca gtagccgcct ctgccgccgc 60

ggagcttccc gaacctcttc agccgcccgg agccgctccc ggagcccggc cgtagaggct 120

gcaatcgcag ccgggagccc gcagcccgcg ccccgagccc gccgccgccc ttcgagggcg 180

ccccaggccg cgccatggtg aaggtgacgt tcaactccgc tctggcccag aaggaggcca 240

agaaggacga gcccaagagc ggcgaggagg cgctcatcat cccccccgac gccgtcgcgg 300

tggactgcaa ggacccagat gatgtggtac cagttggcca aagaagagcc tggtgttggt 360

gcatgtgctt tggactagca tttatgcttg caggtgttat tctaggagga gcatacttgt 420

acaaatattt tgcacttcaa ccagatgacg tgtactactg tggaataaag tacatcaaag 480

atgatgtcat cttaaatgag ccctctgcag atgccccagc tgctctctac cagacaattg 540

aagaaaatat taaaatcttt gaagaagaag aagttgaatt tatcagtgtg cctgtcccag 600

agtttgcaga tagtgatcct gccaacattg ttcatgactt taacaagaaa cttacagcct 660

atttagatct taacctggat aagtgctatg tgatccctct gaacacttcc attgttatgc 720

cacccagaaa cctactggag ttacttatta acatcaaggc tggaacctat ttgcctcagt 780

cctatctgat tcatgagcac atggttatta ctgatcgcat tgaaaacatt gatcacctgg 840

gtttctttat ttatcgactg tgtcatgaca aggaaactta caaactgcaa cgcagagaaa 900

ctattaaagg tattcagaaa cgtgaagcca gcaattgttt cgcaattcgg cattttgaaa 960

acaaatttgc cgtggaaact ttaatttgtt cttgaacagt caagaaaaac attattgagg 1020

aaaattaata tcacagcata accccaccct ttacattttg tgcagtgatt attttttaaa 1080

gtcttctttc atgtaagtag caaacagggc tttactatct tttcatctca ttaattcaat 1140

taaaaccatt accttaaaat ttttttcttt cgaagtgtgg tgtcttttat atttgaatta 1200

gtaactgtat gaagtcatag ataatagtac atgtcacctt aggtagtagg aagaattaca 1260

atttctttaa atcatttatc tggattttta tgttttatta gcattttcaa gaagacggat 1320

tatctagaga ataatcatat atatgcatac gtaaaaatgg accacagtga cttatttgta 1380

gttgttagtt gccctgctac ctagtttgtt agtgcatttg agcacacatt ttaattttcc 1440

tctaattaaa atgtgcagta ttttcagtgt caaatatatt taactattta gagaatgatt 1500

tccaccttta tgttttaata tcctaggcat ctgctgtaat aatattttag aaaatgtttg 1560

gaatttaaga aataacttgt gttactaatt tgtataaccc atatctgtgc aatggaatat 1620

aaatatcaca aagttgttta actagac 1647

<210> 30
<211> 24
<212> DNA
<213> Artificial Sequence

<220>
<223> Description of Artificial Sequence:Artificially
      Synthesized Primer Sequence

<400> 30
atggccgagc cgctccagcc agac 24

<210> 31
<211> 24
<212> DNA
<213> Artificial Sequence

<220>
<223> Description of Artificial Sequence:Artificially
      Synthesized Primer Sequence

<400> 31
tgtttacaga ctactgtcct ctgt 24

## Description

### Technical Field

The present invention relates to genes associated with allergic disease, a method of testing for allergic disease and methods of screening for compounds that serves as candidate therapeutic agents against allergic disease using the expression of the genes as an index.

### Backqround Art

Allergic diseases such as atopic dermatitis are considered to be multifactorial diseases. These diseases are caused by the interaction of many different genes, whose expressions are influenced by several various environmental factors. Thus, determination of specific genes causing a specific disease has been extremely difficult for allergic diseases.

Additionally, expression of mutated or defective genes, or overexpression or reduced expression of specific genes is thought to be involved in allergic diseases. To elucidate the role of gene expression in diseases, it is necessary to understand how a gene is involved in triggering disease onset and how the expression of the gene is altered by external stimulants such as drugs.

Recent developments in gene expression analysis techniques have enabled analysis and comparison of gene expression of many clinical samples. Among these methods, the differential display (DD) method is useful. The differential display method was originally developed by Liang and Pardee in 1992 (Science, 1992, 257: 967-971). According to this method, different samples of several tens or more can be screened at one time to detect genes whose expression are different among the samples. Important information to reveal the causative gene of a disease is expected by examining genes with mutations or genes whose expression changes depending on time and environment. Such genes include those whose expression is influenced by environmental factors.

In recent diagnosis of allergic diseases, generally, history taking, and confirmation of family history and anamnesis of the patient are important factors. Further, methods of diagnosing allergy based on more objective information include a method wherein patient's blood sample are tested and method of observing patient's immune response to allergen. Examples of the former method are the allergen-specific IgE measurement, leukocyte histamine release test, lymphocyte stimulating test, and so on. The presence of allergen-specific IgE verifies the allergic reaction against the allergen. However, allergen-specific IgE is not always detected in every patient. Furthermore, the principle of IgE assay requires performing tests for all of the allergens necessary for diagnosis. Alternatively, the leukocyte histamine release test and lymphocyte stimulating test are methods for observing the reaction of the immune system toward a specific allergen *in vitro*. These methods require complicated operation.

Alternatively, another method wherein the immune response observed in a patient actually contacted with an allergen is utilized in diagnosing allergy is also known (latter method). Such tests include the prick test, scratch test, patch test, intradermal reaction, and induction test. These test methods allow direct diagnosis of patient's allergic reaction, but are invasive tests wherein patients are actually exposed to allergen.

In addition, regardless of the allergen types, methods to testify the involvement of allergic reaction are also attempted. For example, a high serum IgE titer indicates the occurrence of allergic reaction in a patient. The serum IgE titer is the information corresponding to the total amount of allergen-specific IgE. Though it is easy to determine the total amount of IgE regardless of the type of allergen, IgE titer may be reduced in some patients with non-atopic bronchitis and such.

The number of eosinophils and ECP (eosinophil cationic protein) value are items for diagnosing delayed-type reaction following Type I allergy and allergic inflammatory reaction. The number of eosinophils is considered to reflect the advance of allergic symptoms. ECP, a protein contained in eosinophil granules, is also strongly activated in relation to seizures of asthma patients. Even though these diagnostic items reflect allergy symptoms, the scope thereof usable as the diagnostic barometer is limited.

Therefore, diagnostic indicators, regardless of the type of allergen, useful in comprehending pathological conditions of allergic disease patients and for determining the treatment regimen for the disease have been intensely desired in the art. Furthermore, markers for allergic disease that are less harmful to patients and easily provide information required for diagnosis will be of great use.

### Disclosure of the Invention

An objective of the present invention is to provide genes associated with allergic disease. Another objective of the invention is to provide a method of testing for allergic disease and a method of screening for compounds that serve as candidate therapeutic agents for allergic disease using the expression of the genes of the present invention as an index.

Based on a previously established technique, the "Fluorescent differential display method (Fluorescent DD method)" (T. Ito et al. 1994, FEBS Lett. 351: 231-236), the present inventors developed a new DD system wherein T-cell RNA samples prepared from multiple human blood samples can be analyzed. The present inventors applied the DD system to the isolation of genes whose expression level is altered in an allergic disease-specific manner. Specifically, first, the present inventors measured IgE titers against mite antigen in multiple subjects including normal healthy individuals and patients with allergic diseases (bronchial asthma and atopic dermatitis). The results revealed significantly higher IgE titer scores in the allergic disease patient group (hereinafter abbreviated as "patient group" in some cases) than the normal healthy group, confirming the patient group being allergic to mite antigen.

Then, the present inventors divided multiple subjects into normal healthy group and allergic disease patient group, collected T-cells from blood samples of the subjects, and screened genes whose expression level differ between the two groups using the DD system. As a result, the present inventors succeeded in isolating four genes, "B1001," "B1072," "B1151", and "B1466", that showed significantly higher expression levels in the patient group. No involvement of these genes in allergic diseases has been known. Furthermore, the present inventors discovered that allergic disease can be tested and compounds serving as candidate therapeutic agents for allergic disease can be screened using the expression level of these genes as an index, and accomplished the present invention.

Specifically, the present invention relates to genes that show high expression levels in subjects having allergic diathesis, as well as a method of testing for allergic disease and methods of screening for compounds serving as candidate therapeutic agents for allergic disease using the expression level of these genes as an index. More specifically, the present invention relates to a method of testing for allergic disease, a kit for performing the method, and methods of screening for therapeutic agents for allergic disease as follows:
[1] a method of testing for allergic disease, wherein the method comprises the steps of:
   a) measuring the expression level of a gene comprising the nucleotide sequence of any one of SEQ ID NOs: 1, 3, 5, and 6 in T-cells of a subject; and
   b) comparing the expression level of said gene with that in T-cells of a normal healthy subject;
[2] the method of [1], wherein the allergic disease is atopic dermatitis;
[3] the method of [1], wherein the gene expression level is measured by PCR of cDNA;
[4] the method of [1] wherein the gene expression level is measured by detecting a protein encoded by said gene;
[5] a reagent for testing for allergic disease comprising a polynucleotide with a chain length of at least 15 nucleotides and that hybridizes with the nucleotide sequence of any one of SEQ ID NOs: 1, 3, 5, and 6, or a complementary sequence thereto;
[6] a reagent for testing for allergic disease comprising an antibody that recognizes a polypeptide comprising the amino acid sequence of SEQ ID NO: 2 or 4;
[7] a method of screening for compounds serving as candidate therapeutic agents for allergic disease, wherein the method comprises the steps of:
   (1) contacting a candidate compound with a cell that expresses a polynucleotide selected from the group of:
      (a) a polynucleotide comprising a coding region of the nucleotide sequence of any one of SEQ ID NOs: 1, 3, 5, and 6;
      (b) a polynucleotide encoding a protein comprising the amino acid sequence of SEQ ID NO: 2 or 4;
      (c) a polynucleotide encoding a protein comprising the amino acid sequence of SEQ ID NO: 2 or 4 wherein one or more amino acids are substituted, deleted, inserted and/or added, and the expression level of the protein is increased in accordance with allergic disease; and
      (d) a polynucleotide hybridizing under stringent conditions to a DNA consisting of the nucleotide sequence of any one of SEQ ID NOs: 1, 3, 5, and 6, wherein the polynucleotide encodes a protein whose expression level increases in accordance with allergic disease;
   (2) measuring the expression level of the polynucleotide of any one of (a) to (d); and
   (3) selecting the compound that reduces the expression level of said polynucleotide compared to a control;
[8] the method of [7], wherein the cell is a T-cell;
[9] a kit for screening for compounds serving as candidate therapeutic agents for allergic disease, which comprises a polynucleotide having a chain length of at least 15 nucleotides that hybridizes to the nucleotide sequence of any one of SEQ ID NOs: 1, 3, 5, and 6 or a sequence complementary thereto, and a cell that expresses a gene comprising the nucleotide sequence of any one of SEQ ID NOs: 1, 3, 5, and 6;
[10] a kit for screening for compounds serving as candidate therapeutic agents for allergic disease, which comprises an antibody that recognizes a peptide comprising the amino acid sequence of either SEQ ID NO: 2 or 4, and a cell that expresses a gene comprising the nucleotide sequence of either SEQ ID NO: 1 or 3;
[11] an allergic disease animal model consisting of a non-human transgenic vertebrate with an increased expression level in T-cells of the polynucleotide selected from the group of:
   (a) a polynucleotide comprising the coding region of the nucleotide sequence of any one of SEQ ID NOs: 1, 3, 5, and 6;
   (b) a polynucleotide encoding a protein comprising the amino acid sequence of either SEQ ID NO: 2 or 4;
   (c) a polynucleotide encoding a protein comprising the amino acid sequence of either SEQ ID NO: 2 or 4, wherein one or more amino acids are substituted, deleted, inserted and/or added, and the expression level of the protein is increased in accordance with allergic disease; and
   (d) a polynucleotide hybridizing under stringent conditions to a DNA consisting of a nucleotide sequence of any one of SEQ ID NOs: 1, 3, 5, and 6 , wherein the polynucleotide encodes a protein whose expression level is increased in accordance with allergic disease;
[12] a method of screening for compounds serving as candidate therapeutic agents for allergic disease, wherein the method comprises the steps of:
   (1) administering a candidate compound to the animal model of [11] ;
   (2) measuring the expression level of the polynucleotide of any one of (a) to (d) of [11] in T-cells; and
   (3) selecting the compound that reduces the expression level of said polynucleotide compared to a control;
[13] a method of screening for compounds serving as candidate therapeutic agents for allergic disease, wherein the method comprises the steps of:
   (1) contacting a candidate compound with a cell transfected with a vector containing a transcriptional regulatory region of a gene comprising the nucleotide sequence of any one of SEQ ID NOs: 1, 3, 5, and 6, and a reporter gene that is expressed under the control of the transcriptional regulatory region;
   (2) measuring the activity of said reporter gene; and
   (3) selecting the compound that reduces said activity compared to a control;
[14] a therapeutic agent for allergic disease containing a compound that can be obtained by the screening method of any one of claims 7, 12, and 13 as the principal ingredient;
[15] a therapeutic agent for allergic disease containing an antisense DNA of the nucleotide sequence of any one of SEQ ID NOs: 1, 3, 5, and 6, or a portion thereof as the principal ingredient; and
[16] a therapeutic agent for allergic disease containing an antibody that recognizes a peptide comprising the amino acid sequence of either SEQ ID NO: 2 or 4 as the principal ingredient.

The present invention also relates to a method of treating allergic disease comprising any of the steps of:
(a) administering a compound that can be obtained by the screening method of (7), (12) or (13);
(b) administering an antisense DNA of the nucleotide sequence of any one of SEQ ID NOs: 1, 3, 5, and 6, or a portion thereof; and
(c) administering an antibody that recognizes a peptide comprising the amino acid sequence of either SEQ ID NO: 2 or 4.

Furthermore, the present invention relates to the use, in producing a therapeutic agent for allergic disease, of any one of compounds selected from the group of:
(a) a compound that can be obtained by the screening method of (7), (12), or (13);
(b) an antisense DNA for a DNA of the nucleotide sequence of any one of SEQ ID NOs: 1, 3, 5, and 6, or a portion thereof; and
(c) an antibody that recognizes a peptide comprising the amino acid sequence of either SEQ ID NO: 2 or 4.

The present invention relates to a method of testing for allergic disease using the expression levels of "B1001", "B1072", "B1151", and "B1466" in T-cells as an index. All of these genes show altered expression levels between normal healthy group and allergic disease patient group. As described below, the "B1001," "B1072," "B1151", and "B1466" genes of the present invention have been already reported as the SOUL gene (GenBank accession#: AF117616), hsp40 gene (GenBank accession#: D49547), VACM-1 gene (GenBank accession#: X81882), and BRI gene (GenBank accession#: AF152462), respectively. However, the involvement of any of the SOUL gene, hsp40 gene, VACM-1 gene, or BRI gene in allergic disease haven't been reported so far. For the first time, the present inventors have revealed the involvement of these genes in allergic disease.

The genes according to the present invention comprise the nucleotide sequences of SEQ ID NOs listed below, respectively, and amino acid sequences encoded by these nucleotide sequences are those represented by the following SEQ ID NOs:

| | Nucleotide sequence | Amino acid sequence |
|---|---|---|
| "B1001" | SEQ ID NO: 1 | SEQ ID NO: 2 |
| "B1466" | SEQ ID NO: 3 | SEQ ID NO: 4 |
| "B1072" | SEQ ID NO: 5 (partial sequence) | |
| "B1151" | SEQ ID NO: 6 (partial sequence) | |

The sequences of "B1001" and "B1466" are full-length cDNA sequences. Both sequences of "B1072" and "B1151" are partial sequences of full-length cDNAs. The full-length cDNAs containing these partial nucleotide sequences can be obtained by screening T-cell cDNA library using probes that comprise the nucleotide sequence of either SEQ ID NO: 5 and 6. Furthermore, based on the nucleotide sequences of the obtained cDNAs, amino acid sequences encoded by these cDNAs can be deduced. In addition, since the nucleotide sequences of "B1072" and "B1151" are identical to the sequence information previously registered in DNA database, based on the corresponding sequence information, it is also possible to obtain the full-length nucleotide sequences of these cDNAs as well as putative amino acid sequences.

In the present invention, the term "allergic disease" is a general term for diseases in which allergic reaction is involved. More specifically, to consider a disease to be allergic, an allergen must be identified, a strong correlation between the exposure to the allergen and the onset of the pathological change must be demonstrated, and the pathological change must be proven to have an immunological mechanism. Herein, an immunological mechanism means that immune response by the T-cells are induced by the stimulation of the allergen . Examples of allergens include mite antigen, pollen antigen, etc. Representative allergic diseases include bronchial asthma, allergic rhinitis, atopic dermatitis, pollen allergy, insect allergy, and such Allergic diathesis is a genetic factor that is inherited from allergic parents to their children. Familial allergic diseases are also called atopic diseases, and the causative factor that is inherited is the atopic diathesis.

The genes of the present invention, "B1001," "B1072," "131151", and "B1466", showed statistically significant high expression levels in the patient group according to the comparison between the normal healthy subject group and allergic disease patient group. Therefore, it is possible to test for allergic disease using the expression level of "B1001," "B1072," "B1151," or "B1466" as an index.

Tests for allergic disease of the present invention include, for example, those as described below. A test for judging whether an allergic disease-like symptom is caused by allergic reaction can be mentioned. More specifically, allergic disease-like symptoms are exemplified by dermatitis (itching, flare); rhinitis (nasal congestion, running nose, sneeze); asthma (stridor, dyspnea); etc. Although these symptoms are also observed in xeroderma, cold syndrome (cold in the nose), bronchitis, and such, it is possible to judge whether these symptoms are caused by allergic reaction or not according to the test method of the present invention. In addition, the method of testing for allergic disease of the present invention includes a test to judge whether a subject has allergic diathesis or not.

Herein, the expression levels of the genes "B1001", "B1072", "B1151", and "B1466" include the transcription of the genes to mRNAs as well as the translation into proteins. Therefore, a method for testing for allergic disease according to the present invention is performed by comparing the expression intensity of mRNA corresponding to the gene, or the expression level of a protein encoded by the gene.

Measurement of the expression levels of the genes "B1001", "B1072", "B1151", and "B1466" in a test for allergic disease of the present invention may be conducted according to known gene analytical methods. More specifically, for example, a hybridization technique with a nucleic acid that hybridizes to respective genes as a probe, a gene amplification technique with a DNA hybridizing to the gene of this invention as a primer, or such can be utilized.

Polynucleotides having a chain length of at least 15 nucleotides and that specifically hybridize to a gene of the present invention are used as probes and primers in the test of the present invention. Herein, the term "specifically hybridize" means that, under normal hybridization conditions, preferably under stringent hybridization conditions, no significant cross hybridization occur with DNA and/or RNA encoding other genes. A stringent condition can be provided, for example, by hybridizing a probe to a transfer membrane at 68°C in Express Hybridization Solution (Clontech), and finally, washing in 0.1x SSC, 0.05% SDS at 50°C.

A "polynucleotide" of the present invention may be either DNA or RNA. The polynucleotides may be either synthetic or naturally-occurring. A DNA used as a probe for hybridization is usually labeled. Examples of labeling methods include:
· nick translation labeling using DNA polymerase I;
· end labeling using polynucleotide kinase;
· fill-in end labeling using Klenow fragment (Berger SL, Kimmel AR (1987) Guide to Molecular Cloning Techniques, Method in Enzymology, Academic Press; Hames BD, Higgins SJ (1985) Genes Probes: A Practical Approach. IRL Press; Sambrook J, Fritsch EF, Maniatis T. (1989) Molecular Cloning: a Laboratory Manual, 2nd Ed. Cold Spring Harbor Laboratory Press) ;
· transcription labeling using RNA polymerase (Melton DA, Krieg PA, Rebagkiati MR, Maniatis T, Zinn K, Green MR. (1984) Nucleic Acids Res., 12, 7035-7056); and
·non-isotopic labeling of DNA by incorporating modified nucleotides (Kricka LJ. (1992) Nonisotopic DNA Probing Techniques. Academic Press).

The test for allergic disease using hybridization techniques may be conducted utilizing, for example, Northern hybridization, dot blot hybridization, or DNA microarray technique. Furthermore, gene amplification techniques, such as RT-PCR method may be used. The use of the PCR amplification monitoring method during the gene amplification step in RT-PCR enables a more quantitative analysis of the gene expression of the present invention.

In the PCR gene amplification monitoring method, probes that are dual-labeled at both ends with different fluorescent dyes whose fluorescences quench each other is hybridized to the detection target (DNA or reverse transcript of RNA). With the progress of PCR, the 5'-3' exonuclease activity of Taq polymerase degrades the probe to separate the two fluorescent dyes from each other, and their fluorescence can be detected. The fluorescence is detected at real time. By simultaneously conducting a measurement using a standard sample with a known copy number, it is possible to determine the copy number of the target in the subject sample at the cycle number where PCR amplification is linear (Holland, P. M. et al., 1991, Proc. Natl. Acad. Sci. USA 88: 7276-7280; Livak, K. J. et al., 1995, PCR Methods and Applications 4(6): 357-362; Heid, C. A. et al., 1996, Genome Research 6: 986-994; Gibson, E. M. U. et al., 1996, Genome Research 6: 995-1001). For the PCR amplification monitoring method, for example, ABI PRISM7700 (PE Biosystems) may be used.

The method for testing for allergic disease of the present invention can be also carried out by detecting a protein encoded by the gene "B1001", "B1072", "B1151", or "131466". For such test methods , for example, the Western blotting method, the immunoprecipitation method, the ELISA method, and such that utilize antibodies binding to a protein encoded by these genes may be employed.

Antibodies that bind to "B1001", "B1072", "B1151", or "B1466" proteins used in the detection may be produced by techniques known to those skilled in the art. Antibodies used in the present invention may be polyclonal or monoclonal antibodies (Milstein, C. et al., 1983, Nature 305 (5934) : 537-40). For example, polyclonal antibody against a protein of the present invention may be produced by collecting blood from mammals sensitized with an antigen, and separating the serum from this blood using known methods. As a polyclonal antibody, the serum containing polyclonal antibody may be used. According to needs, a fraction containing polyclonal antibody can be further isolated from this serum. Alternatively, a monoclonal antibody can be obtained by isolating immune cells from mammals sensitized with an antigen; fusing these cells with myeloma cells, and such; cloning hybridomas thus obtained; and collecting the antibody as the monoclonal antibody.

To detect "B1001", "B1072", "B1151", and "B1466" proteins, these antibodies may be appropriately labeled. Alternatively, instead of labeling the antibody, a substance that specifically binds to antibodies, for example, protein A or protein G, may be labeled to arrange an indirect detection of the proteins. More specifically, one example of an indirect detection method is ELISA.

A protein or partial peptides thereof that is used as an antigen may be obtained, for example, by inserting a gene or portion thereof into an expression vector, introducing it into an appropriate host cell to produce a transformant, culturing the transformant to express the recombinant protein, and purifying the expressed recombinant protein from the culture or the culture supernatant. Alternatively, oligonucleotides consisting of the amino acid sequences encoded by these genes (SEQ ID NOs: 2, and 4), or partial amino acid sequences of the amino acid sequence encoded by the full-length cDNA that can be obtained based on SEQ ID NOs: 5 and 6 are chemically synthesized to be used as the antigen.

T-cells from subjects are used as the test sample in the present invention. T-cells can be prepared from peripheral blood by known methods. Specifically, for example, heparinized collected blood is fractionated by centrifugation to isolate lymphocytes. The separated lymphocytes may be directly used as the sample for the test for allergic disease of the present invention. Direct analysis of not a purified T-cell fraction but the lymphocyte fraction as a test sample enables a convenient bed-side test. Alternatively, T-cells may be isolated by fractionating CD3-positive cells from separated lymphocytes using CD3 microbeads labeling, followed by separation using a cell sorter, and such. Lysate prepared by disintegrating the separated T-cells may serve as a sample for the immunological assay of the above-described protein. Alternatively, mRNA extracted from this lysate may be used as a sample for measuring mRNA corresponding to the gene. Preparation of T-cell lysate and mRNA extraction may be conveniently carried out using commercially available kits.

Alternatively, in the case where the protein to be detected is a secretory protein, comparison of the expression level of a gene encoding the protein is accomplished by measuring the amount of the target protein contained in body fluid sample, such as blood and serum, in a subject.

When the expression level of a gene of the present invention is higher in a subject compared with that in normal healthy individuals as a result of testing for allergic disease according to the present invention, the subject may be determined to suffer allergic disease. Alternatively, in the test for an allergic diathesis, the subject may be judged to have allergic diathesis.

Furthermore, the present invention relates to an allergic disease animal model comprising a non-human transgenic animal with an increased expression level in T-cells of a polynucleotide selected from the group of:
(a) a polynucleotide comprising a coding region of the nucleotide sequence of any one of SEQ ID NOs: 1, 3, 5, and 6;
(b) a polynucleotide encoding a protein comprising the amino acid sequence of SEQ ID NO: 2 or 4;
(c) a polynucleotide encoding a protein comprising the amino acid sequence of SEQ ID NO: 2 or 4 wherein one or more amino acids are substituted, deleted, inserted and/or added, and the expression level of the protein is increased in accordance with allergic disease; and
(d) a polynucleotide hybridizing under stringent conditions to a DNA consisting of the nucleotide sequence of any one of SEQ ID NOs: 1, 3, 5, and 6, wherein the polynucleotide encodes a protein whose expression level increases in accordance with allergic disease.

According to the present invention, the expression levels of the genes "B1001," "B1072," "B1151" and "B1466" were revealed to be elevated in T-cells in accordance with allergic disease. Therefore, an animal wherein the expression levels in T-cells of these genes or genes functionally equivalent thereto are artificially increased can be used as an allergic disease animal model. Herein, the term "functionally equivalent gene" refers to any one of the polynucleotides (a) to (d) described above. Typical examples of the functionally equivalent genes include those having different nucleotide sequences generated by polymorphism, splicing isoforms, and such. Counterparts of the "B1001," "B1072," "B1151" and "B1466" genes can be obtained by PCR cloning and hybridization screening based on the nucleotide sequence information disclosed in the present invention. In the case where the expression level of the obtained counterpart of each gene increases in accordance with allergic disease, the gene is confirmed to be a functionally equivalent gene. Animal models of the present invention can be obtained, for example, by known methods as follows.

More specifically, a transgenic animal can be obtained by a method wherein the gene and ovum are mixed and treated with calcium phosphate; a method wherein the gene is introduced directly into the nucleus of oocyte in pronuclei with a micropipette under a phase contrast microscope (the microinjection method, US Patent No. 4873191); or a method wherein embryonic stem cells (ES cells) are used. Furthermore, methods have been developed wherein ovum is infected with a gene-inserted retrovirus vector; the sperm vector method wherein a gene is transduced into ovum via sperm; and such. The sperm vector method is a gene recombination technique for introducing a foreign gene by fertilizing ovum with sperm after incorporating a foreign gene into the sperm by adhesion, electroporation method, etc. (M. Lavitranoet, et al., Cell, 57, 717, 1989).

Transgenic animals of the present invention are useful in not only screening for drugs for treating or preventing allergic diseases as described below but also are useful for elucidating mechanisms of allergic diseases, as well as testing the safety of the screened compounds.

Furthermore, the present invention relates to a method of screening for compounds serving as candidate therapeutic agents for allergic disease. Herein, the expression level of genes, "B1001" gene, "B1072" gene, "B1151" gene, and "B1466" gene, are significantly increased in human suffering from allergic disease. Therefore, therapeutic agents for allergic disease may be obtained by selecting compounds reducing the expression level of these genes. A compound that reduces the expression level of a gene of the present invention has an inhibitory effect on any step including transcription of the gene, translation of the gene, or activity expression of a protein.

A method of screening for compounds serving as candidate therapeutic agents for allergic disease of the present invention can be carried out either *in vivo* or *in vitro*. For *in vivo* screening, an allergic disease animal model comprising a non-human transgenic animal wherein a gene of any one of the above-described (a) to (d) is highly expressed in T-cells can be used. The screening can be carried out, for example, following the steps of:
(1) administering a test compound to an allergic disease animal model, a non-human transgenic animal wherein the expression in T-cells of a polynucleotide of any one of the above-described (a) to (d) is increased;
(2) measuring the expression level of the polynucleotide of any of the above-described (a) to (d) in the T-cells of allergic disease animal model; and
(3) selecting the compound that reduces the expression level of the polynucleotide compared to a control.

For *in vitro* screening, for example, a method wherein a candidate compound is contacted with cells expressing a gene of any one of the above-descried (a) to (d) , and selecting the compound that reduces the expression level of the gene can be mentioned. The screening can be carried out, for example, following the steps of:
(1) contacting a candidate compound with cells that express a polynucleotide of any one of the above-described (a) to (d) ;
(2) measuring the expression level of the polynucleotide of any one of the above-described (a) to (d); and
(3) selecting the compound that reduces the expression level of the polynucleotide compared to a control.

According to the present invention, cells expressing a polynucleotide of any one of the above-described (a) to (d) can be obtained by inserting the polynucleotide into an appropriate expression vector, and transfecting the vector into a suitable host cell. Any vectors and host cells may be used, so long as they express a gene of this invention. Examples of host cells in the host-vector system include *Escherichia coli,* yeast, insect cells, animal cells, etc., and vectors for respective host cells can be appropriately selected.

Vectors may be transfected into the host by biological methods, physical methods, chemical methods, and so on. Examples of the biological methods include methods using virus vectors; methods using specific receptors; and the cell-fusion method (HVJ (Sendai virus) method, the polyethylene glycol (PEG) method, the electric cell fusion method, and microcell fusion method (chromosome transfer)). Examples of the physical methods include the microinjection method, the electroporation method, and the method using gene particle gun. The chemical methods are exemplified by the calcium phosphate precipitation method, the liposome method, the DEAE-dextran method, the protoplast method, the erythrocyte ghost method, the erythrocyte membrane ghost method, and the microcapsule method.

Screening for compounds that serve as candidate therapeutic agents for allergic disease of the present invention can be also carried out using T-cell lines. Examples of T-cell lines include Molt-4 cell and Jurkat cell. According to this screening method, first a candidate compound is added to the T-cell line. Then, the expression level of a polynucleotide of any one of the above-described (a) to (d) is measured in the T-cell line to select the compound that reduces the expression level of the polynucleotide.

According to the screening method of this invention, the expression levels of any one of the polynucleotides of the above-described (a) to (d) can be compared not only by detecting the expression levels of proteins encoded by these genes but also by detecting corresponding mRNAs. To compare the expression levels using mRNA, the process for preparing mRNA sample as described above is carried out in place of the process for preparing protein samples. Detection of mRNA and protein can be performed by known methods as described above.

Candidate test compounds used in such screening include, besides compound preparations synthesized by existing chemical methods, such as steroid derivatives, and compound preparations synthesized by combinatorial chemistry; mixtures of multiple compounds, such as extracts from animal or plant tissues, and microbial cultures; preparations purified thereof; and so on.

Furthermore, based on the disclosure of this invention, the transcriptional regulatory region of a gene of the present invention can be obtained to construct a reporter assay system. The term "reporter assay system" refers to an assay system for screening for a transcriptional regulatory factor that acts on the transcriptional regulatory region using the expression level of a reporter gene localized downstream of the transcriptional regulatory region as an index.

A transcriptional regulatory region is exemplified by promoter, enhancer, as well as CAAT box, TATA box, and such, that are usually found in the promoter region. As a reporter gene, the CAT (chloramphenicol acetyltransferase) gene, the luciferase gene, growth hormone genes, and such can be utilized.

A transcriptional regulatory region of a gene of the present invention can be obtained as follows. Specifically, first, based on the nucleotide sequence of a cDNA disclosed in this invention, a human genomic DNA library, such as BAC library and YAC library, is screened by a method using PCR or hybridization to obtain a genomic DNA clone containing the sequence of the cDNA. Based on the sequence of the resulting genomic DNA, the transcriptional regulatory region of a cDNA disclosed in this invention is predicted and obtained. The obtained transcriptional regulatory region is cloned so as to be localized upstream of a reporter gene to prepare a reporter construct. The resulting reporter construct is introduced into a cultured cell strain to prepare a transformant for screening. By contacting the transformant with a candidate compound, the compound that controls the expression of the reporter gene can be screened.

The polynucleotide, antibody, cell strain, or animal model necessary for the various screening methods according to this invention can be previously combined as a kit. A substrate compound used for the detection of a label, medium and vessel for cell culturing, positive and negative standard samples, further a direction of the kit may be also packaged in these kits.

A compound selected by the screening of the present invention is useful as a therapeutic agent for allergic disease. Alternatively, an antisense DNA that suppresses the expression of a gene comprising the nucleotide sequence of any one of SEQ ID NOs: 1, 3, 5, and 6 is also useful as a therapeutic agent for allergic disease. Furthermore, an antibody that recognizes a protein encoded by the nucleotide sequence of any one of SEQ ID NOs: 1, 3, 5, and 6 is useful as a therapeutic agent for allergic disease.

Genes comprising the nucleotide sequence of any one of SEQ ID NOs: 1, 3, 5, and 6 are genes whose expression level is elevated in T-cells of patients with allergic disease. Therefore, suppression of the expression of these genes or suppression of the function of proteins encoded by these genes is expected to have therapeutic effect on allergic disease.

A therapeutic agent for allergic disease of the present invention can be formulated by including a compound selected by the screening methods as the effective ingredient, and mixing with a physiologically acceptable carrier, excipient, diluent, and such. Aiming at the amelioration of allergic symptoms , the therapeutic agent for allergic disease of this invention can be administered orally or parenterally.

Oral drugs can take any dosage forms selected from the group of granule, powder, tablet, capsule, solution, emulsion, suspension, and so on. Injections can include subcutaneous injection, intramuscular injection, intraperitoneal injection, and so on.

Furthermore, for administering a compound that is composed of protein, a therapeutic effect can be achieved by introducing a gene encoding the protein into the living body using gene therapeutic techniques. The techniques for treating disease by introducing a gene encoding a therapeutically effective protein into the living body and expressing it therein are known in the art.

Alternatively, an antisense DNA can be incorporated downstream of an appropriate promoter sequence to be administered as an antisense RNA expression vector. When this expression vector is introduced into T cells of an allergic disease patient, the therapeutic effect on allergic disease is achieved by the reduction of the expression level of the gene through the expression of the corresponding antisense gene. For introducing the expression vector into T cells, methods performed either *in vivo* or *ex vivo* are known.

Although the dosage may vary depending on the age, sex, body weight, and symptoms of a patient; treatment effects; method for administration; treatment duration; type of active ingredient contained in the drug composition; and such, a range of 0.1 to 500 mg, preferably 0.5 to 20 mg per dose for an adult can be administered. However, the dose changes according to various conditions, and thus in some case a more smaller amount than that mentioned above is sufficient whereas an amount above the above-mentioned range is required in other cases.

All the literatures for the prior arts cited in the present specification are herein incorporated by reference.

### Brief Description of the Drawings

Fig. 1 shows the distribution of the "B1001" expression level among subjects divided into normal healthy group and patient group (patients with asthma and patients with atopic dermatitis).
Fig. 2 shows the distribution of the "B1072" expression level among subjects divided into normal healthy group and patient group (patients with asthma and patients with atopic dermatitis).
Fig. 3 shows the distribution of the "B1151" expression level among subjects divided into normal healthy group and patient group (patients with asthma and patients with atopic dermatitis).
Fig. 4 shows the distribution of the "B1466" expression level among subjects divided into normal healthy group and patient group (patients with asthma and patients with atopic dermatitis).
Fig. 5 shows the distribution of the "B1072" expression level among subjects divided into three groups, i.e., normal healthy group, asthma patient group, and atopic dermatitis patient group.
Fig. 6 shows the distribution of the "B1151" expression level among subjects divided into three groups, i.e., normal healthy group, asthma patient group, and atopic dermatitis patient group.
Fig. 7 depicts gel electrophoretograms showing the results of Northern hybridization of the "B1001" gene of the present invention. The left panel shows the results on various immune-associated tissues, and the right panel shows those on cancer cell lines. Each lane represents respective tissue or cell line as follows:
   Left gel: (I) spleen, (II) lymph node, (III) thymus, (IV) peripheral blood leukocyte, (V) bone marrow, and (VI) fetal liver.
   Right gel: (I) promyelocytic leukemia HL-60, (II) HeLa cell S3, (III) chronic myelocytic leukemia K-562, (IV) lymphoblastic leukemia MOLT-4, (V) Burkitt's lymphoma Raji, (VI) colorectal adenocarcinoma SW480, (VII) lung carcinoma A549, and (VIII) melanoma G361.

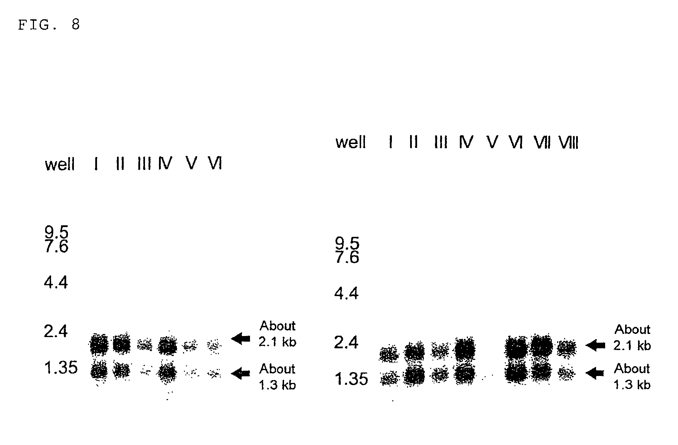

Fig. 8 depicts gel electrophoretograms showing the results of Northern hybridization of the "B1466" gene of the present invention. The left panel shows the results on various immune-related tissues, and the right panel shows those on cancer cell lines. Each lane represents respective tissue or cell line as follows:
   Left gel: (I) spleen, (II) lymph node, (III) thymus, (IV) peripheral blood leukocyte, (V) bone marrow, and (VI) fetal liver.
   Right gel: (I) promyelocytic leukemia HL-60, (II) HeLa cell S3, (III) chronic myelocytic leukemia K-562, (IV) lymphoblastic leukemia MOLT-4, (V) Burkitt's lymphoma Raji, (VI) colorectal adenocarcinoma SW480, (VII) lung carcinoma A549, and (VIII) melanoma G361.

### Best Mode for Carrying out the Invention

The present invention will be explained in more detail below with reference to examples, but is not to be construed as being limited thereto.

### [Example 1] Collection of blood samples from patients and normal healthy subjects

To isolate genes that show different expression in an allergic disease-specific manner, blood samples were collected from patients and normal healthy volunteers selected after analysis of their symptoms. The blood samples were collected as a group of normal healthy subjects and patients with a very mild case of allergy, a group of patients with bronchial asthma, and a group of patients with atopic dermatitis from 12, 23 and 24 people, respectively. The amount of mite-antigen-specific IgE was measured using a part of the blood samples.

The specific IgE measurement was conducted according to the CAP radioallergosorbent test (CAP RAST) method, a modified RAST method, that uses a paper disk as a solid phase. Serum from Pharmacia, which has a standard antibody titer, was used as the standard to determine IgE antibody titers in respective samples. The obtained values were scored.

Scores of mite-specific IgE antibody titers of each subject are shown in the column under the title of "mite-specific IgE antibody score" of Table 1. As shown in the table, the scores in the group of normal healthy subjects and patients of very mild case of allergy were "0" except for one patient of a very mild case. On the other hand, the patient group showed high scores, indicating that patients in this group are allergic toward the mite antigen.

**Table 1**

| Subject No. | Disease | Mite antigen specific IgE score | B1072 | B1151 | B1001 | B1466 |
|---|---|---|---|---|---|---|
| 01 | Very mild | 0 | . | . | 506 | 9,828 |
| 02 | Very mild | 0 | 9,053 | . | 1,221 | 8,409 |
| 03 | Very mild | 5 | 13,425 | . | 573 | 6,433 |
| 04 | Very mild | 0 | 21,118 | 1,794 | 1,004 | 7,297 |
| 05 | Very mild | 0 | 7,607 | 1,089 | 2,927 | 11,922 |
| 06 | Very mild | 0 | 14,149 | 1,088 | 700 | 13,662 |
| 07 | Normal | 0 | 7,608 | 417 | 1,633 | 12,399 |
| 08 | Normal | 0 | 10,115 | 839 | 2,777 | 5,263 |
| 09 | Normal | 0 | 8,036 | 544 | 2,467 | 13,317 |
| 10 | Normal | 0 | 7,644 | 876 | 1,884 | 10,882 |
| 11 | Normal | 0 | 7,044 | 795 | 1,506 | 13,683 |
| 12 | Normal | 0 | . | . | 679 | 6,455 |
| 13 | Asthma | 5 | 20,213 | . | 977 | 5,645 |
| 14 | Asthma | 5 | 10,530 | 1,239 | 518 | 5,619 |
| 15 | Asthma | 4 | . | . | . | . |
| 16 | Asthma | 5 | 19,467 | 1,585 | 1,034 | 6,979 |
| 17 | Asthma | 5 | 11,254 | 1,454 | 3,973 | 14,409 |
| 18 | Asthma | 5 | 10,573 | 1,580 | 1,040 | 8,376 |
| 19 | Asthma | 6 | 10,571 | 557 | 1,066 | 7,991 |
| 20 | Asthma | 6 | 9,016 | 960 | 1,469 | 10,597 |
| 21 | Asthma | 4 | 9,495 | 982 | 4,799 | 18,518 |
| 22 | Asthma | 5 | 7,016 | 762 | 1,633 | 9,604 |
| 23 | Asthma | 5 | 12,140 | 788 | 1,855 | 16,034 |
| 24 | Asthma | 6 | 18,495 | 2,587 | 1,247 | 7,711 |
| 25 | Asthma | 5 | 9,533 | 1,031 | 1,854 | 14,225 |
| 26 | Asthma | 6 | 5,515 | 646 | 1,740 | 12,451 |
| 27 | Asthma | 5 | 17,331 | 2,973 | 1,068 | 6,173 |
| 28 | Asthma | 6 | 13,357 | 2,270 | 1,105 | 11,213 |
| 29 | Asthma | 6 | . | . | . | . |
| 30 | Asthma | 6 | 8,901 | 1,506 | 1,164 | 15,518 |
| 31 | Asthma | 4 | . | . | . | . |
| 32 | Asthma | 4 | 6,450 | 1,177 | 2,078 | 18,213 |
| 34 | Asthma | 4 | 25,285 | 3,999 | 2,471 | 14,427 |
| 35 | Asthma | 6 | 13,276 | 2,511 | 2,634 | 13,531 |
| 36 | Asthma | 5 | 15,689 | 2,025 | 1,744 | 16,219 |
| 37 | Dermatitis | 3 | 18,283 | 3,316 | 4,123 | 20,577 |
| 38 | Dermatitis | 4 | . | . | 877 | 6,327 |
| 39 | Dermatitis | 5 | 44,227 | 3,801 | 1,822 | 14,085 |
| 40 | Dermatitis | 4 | 30,564 | 4,311 | 1,977 | 10,640 |
| 41 | Dermatitis | 6 | 10,556 | 2,512 | 5,712 | 23,206 |
| 42 | Dermatitis | 6 | 14,357 | 1,991 | 1,722 | 12,038 |
| 43 | Dermatitis | 6 | 45,884 | 2,024 | 1,111 | 9,891 |
| 44 | Dermatitis | 5 | 16,621 | 1,572 | 1,935 | 11,647 |
| 45 | Dermatitis | 6 | 16,000 | 1,703 | 1,007 | 11,792 |
| 46 | Dermatitis | 6 | 6,553 | 1,082 | 6,303 | 30,759 |
| 47 | Dermatitis | 5 | 8,333 | 2,121 | 5,000 | 22,896 |
| 48 | Dermatitis | 3 | 47,906 | 12,291 | 1,605 | 8,878 |
| 49 | Dermatitis | 6 | 10,917 | 2,003 | 776 | 7,675 |
| 50 | Dermatitis | 6 | . | . | 936 | 14,514 |
| 51 | Dermatitis | 6 | . | . | 2,744 | 15,965 |
| 52 | Dermatitis | 6 | . | . | 2,600 | 12,241 |
| 53 | Dermatitis | 5 | 14,593 | 1,974 | 1,675 | 10,802 |
| 54 | Dermatitis | 6 | 14,841 | 2,291 | 843 | 11,785 |
| 55 | Dermatitis | 4 | 11,774 | 1,675 | 762 | 9,816 |
| 56 | Dermatitis | 6 | . | . | . | . |
| 57 | Dermatitis | 5 | 15,004 | 1,972 | . | . |
| 58 | Dermatitis | 5 | 11,810 | 2,472 | 1,406 | 7,604 |
| 59 | Dermatitis | 5 | 11,377 | 1,661 | 1,425 | 7,497 |
| 60 | Dermatitis | 6 | 11,415 | 1,693 | 1,670 | 7,320 |

### [Example 2] Preparation of lymphocyte fractions from blood samples

T-cells were prepared from 10 ml blood sample as follows. First, 1 ml heparin (purchased from Novo Co., etc.) was thoroughly spread over the 10 ml-syringe wall surface, and then 10 ml blood sample including a final concentration of 50 units/ml heparin was collected. For blood collection, two 22G needles for each person were prepared. After removing the needle from the syringe, the blood sample was transferred to a 50-ml centrifuge tube (polypropylene). The tube was centrifuged at 1500 rpm for 5 min at room temperature and then 1.1 ml was taken from as close to the surface as possible. After further 15000 rpm centrifugation for 5 min at 4°C, 1 ml of the supernatant was collected as plasma. An equal amount (9 ml) of 0.9% NaCl containing 3% dextran (Nacalai) was added to the remaining sample. This mixture was inverted gently several times, and then was left standing for 30 min at room temperature. PRP (platelet rich plasma) was transferred to a new 15 ml centrifuge tube and centrifuged at 1200 rpm (equivalent to 150x g for the Tomy centrifuge) for 5 min at room temperature. After the centrifugation, platelets were present in the supernatant. Precipitated cells were resuspended in 5 ml Ca and Mg-free HBSS (GIBCO, etc.). The cell suspension was layered on the top of a 5 ml Ficoll Paque (Pharmacia) -containing Falcon tube (2006 or 2059, polypropylene) with a capillary pipette. After centrifuging the tube at 1200 rpm for 5 min, it was further centrifuged at 1500 rpm (equivalent to 400x g for the Tomy centrifuge) for 30 min at room temperature. As a result, granulocytes and erythrocytes were precipitated, and lymphocytes, monocytes, and platelets were included in the middle layer, with the Ficoll layer between the precipitate and the middle layer.

The middle layer was collected using a Pasteur pipette. Two to three volumes of bovine serum albumin (BSA)/phosphate buffered saline (PBS) (0.5% BSA, 2 mM EDTA in PBS, pH 7.2, degassed just before use) were added thereto, and the mixture was centrifuged at 1200 rpm for 5 min at 4°C. The precipitate was collected and washed twice with BSA/PBS solution. After the second wash, cells were resuspended in 5 ml BSA/PBS, and a portion of the supernatant was diluted two-fold with trypan blue to count the cell number. Total cell numbers were about 1x 10⁷, and the suspension was used as lymphocyte fraction.

### [Example 3] T-cell separation from lymphocyte fraction

The lymphocyte fraction obtained in Example 2 was centrifuged at 1200 rpm for 5 min at 4°C, and the precipitate was resuspended in BSA/PBS at 10⁸ cells/100 µl. The volume was approximately 20 µl. The cell suspension was transferred to an Eppendorf tube (1.5 ml), and then CD3 microbead solution was added thereto. This sample was allowed to stand at 4 to 10°C for 30 min (not on ice) and was further treated using magnetic cell sorter (MACS, Miltenyi Biotech Inc.) by the following procedure.

An MS⁺/RS⁺ column was set on Mini MACS or Vario MACS separation unit (without needles) . 500 µl of BSA/PBS was gently applied onto the column, and the buffer was run off. Then CD3 microbead-labeled cells were applied onto the column. The column was washed three times with 500 µl BSA/PBS (B-cell fraction) . The column was detached from the separation unit and set onto a tube to collect the eluate. 1 ml of BSA/PBS was applied onto the column, and CD3-positive cells were eluted rapidly using a plunger attached to the column. The eluate was used as T-cell fraction.

The obtained T-cell fraction was centrifuged at 1200 rpm at 4°C for 5 min. The precipitate was washed twice with BSA/PBS. After the second wash, the cells were resuspended in 1 ml BSA/PBS, and a portion of the suspension was diluted two-fold with trypan blue to count the cell number. Total cell numbers were approximately 4x 10⁶.

### [Example 4] Total RNA preparation from T-cells

Total RNA was prepared from T-cells using RNeasy Mini (Qiagen) basically following the manufacturers' instruction. All manipulations were carried out at room temperature, wearing gloves. Four-fold volume of ethanol was added to the wash buffer RPE. To the lysis buffer RLT, 10 µl/ml of 2-mercaptoethanol was added.

The cell suspension was centrifuged at 1000 to 1200 rpm for 5 min, and the supernatant was removed by aspiration. The precipitate was resuspended in 350 µl lysis buffer RLT (containing 2-mercaptoethanol). At this step, the cell lysate in the lysis buffer RLT could be stored at -70°C. The frozen stored cell lysate was thawen by incubation at 37°C for 10 to 15 min, and, if insoluble matter was observed, was centrifuged for 3 min at maximum speed to collect the supernatant alone. The lysate was homogenized by syringe with a 20G Cathelin needle, and then 350 µl lysate was applied onto QIA shredder with a Pipetman, and centrifuged at 1500 rpm for 2 min to collect the eluate. 350 µl of 70% ethanol was added thereto and mixed well by pipetting. An RNeasy spin column was fixed to the attached 2-ml tube, and the lysate mixture was applied onto the column. The column was centrifuged at 8000x g (11500 rpm) for 1 min, and the flow through was discarded. Then 700 µl wash buffer RW1 was applied onto the column, and the column was left standing capped for 5 min. The column was centrifuged at 11500 rpm for 15 seconds, and the flow through was discarded. The column was attached to a new 2-ml tube, 500 µl wash buffer RW1 was applied onto the column, centrifuged at 11500 rpm for 15 min, and the flow through was discarded. 500 µl wash buffer RPE was applied onto the column, and centrifuged at full speed for 2 min. The column was attached to a new tube (1.5 ml), 30 µl of DEPC treated water was applied thereto, and the capped column was allowed to stand for 10 min. The column was centrifuged at 11500 rpm for 10 min to obtain total RNA. The concentration of the RNA was measured. If the amount was low, the column was set again onto a new 1.5-ml tube, and 30 µl of DEPC treated water was applied thereto. Then the column was left standing capped for 10 min, and centrifuged at 11500 rpm for 10 min to obtain total RNA.

### [Example 5] DNase treatment of total RNA

In order to remove DNA from the total RNA prepared from the T-cells, DNase treatment was performed. The treatment was conducted in a reaction mixture containing 2 units of DNase (Nippon Gene) and 50 units of RNase inhibitor (Pharmacia) in 100 µl of 1x DNase buffer (Nippon Gene) . After incubating this mixture at 37°C for 15 min, an equal volume of PCI (phenol: chloroform: isoamyl alcohol = 25:24:1) was added thereto, and the tube was vortexed. The tube was centrifuged at 12000 rpm at room temperature for 10 min, and the upper phase (aqueous phase) was transferred to a new 1.5-ml tube . One tenth volume of 3 M sodium acetate (pH 5.2) and 2.5 volumes of 100% ethanol with 1 µl of Ethachinmate were added thereto, and the mixture was inverted several times. After allowing the tube to stand at -20°C for 15 min, it was centrifuged at 12000 rpm for 15 min at 4°C. The supernatant was removed, and 70% ethanol was added to the precipitate. After tapping the tube until the precipitate was detached from the tube, the supernatant was completely removed. The precipitate was dried for 3 min and dissolved in 10 to 20 µl DDW (DNase and RNase free). The concentration was measured, and the sample was stored at -80°C until use.

### [Example 6] Differential Display (DD) analysis using total RNA prepared from T-cells

Fluorescent Differential Display (abbreviated to DD) analysis using total RNA prepared from T-cells was carried out according to the literature (T. Ito et al., 1994, FEBS Lett. 351: 231-236). The total RNA prepared from the T-cells was reverse transcribed to obtain cDNA. In the first DD-PCR reaction, 0.2 µg each of total RNA was used for three types of anchor primers to synthesize cDNAs. In the second DD-PCR reaction, 0.4 µg each of total RNA was used for the synthesis of cDNAs using three types of anchor primers, respectively. In both cases, the cDNAs were diluted to a final concentration equivalent to 0.4 ng/µl RNA and used for further experiments . The DD-PCR reaction was carried out using an amount of cDNA equivalent to 1 ng RNA per reaction. The reaction mixture composition was as shown in Table 2.

**Table 2**

| | |
|---|---|
| cDNA (equivalent to 0.4 ng/µl RNA) | 2.5 µl |
| Arbitrary primer (2 µM) | 2.5 µl |
| 10x AmpliTaq PCR buffer | 1.0 µl |
| 2.5 mM dNTP | 0.8 µl |
| 50 µM anchor primer (GT15A, GT15C, or GT15G) | 0.1 µl |
| Gene Taq (5 U/µl) | 0.05 µl |
| AmpliTaq (5 U/µl) | 0.05 µl |
| dH₂O | 3.0 µl |
| Total volume | 10.0 µl |

The PCR reaction was carried out at following condition: 1 cycle of "95°C for 3 min, 40°C for 5 min, and 72°C for 5 min"; subsequently 30 cycles of "94°C for 15 sec, 40°C for 2 min, and 72°C for 1 min"; after these cycles, 72°C for 5 min; and then continuously 4°C.

Reactions were conducted using 287 primer pairs: i.e., anchor primers GT15A (SEQ ID NO: 7), GT15C (SEQ ID NO: 8), and GT15G (SEQ ID NO: 9) were used in combination with arbitrary primers AG 1 to AG 110, AG 111 to AG 199, and AG 200 to AG 287, respectively. As for the arbitrary primers, oligomers having 10 nucleotides with a GC content of 50% were designed and synthesized.

For gel electrophoresis, a 6% denaturing polyacrylamide gel was prepared, and 2.5 µl sample from the PCR was applied and run under 40 W for 210 min. After electrophoresis, the gel was scanned by Hitachi fluorescence imaging analyzer FMBIO II, and the gel image was obtained by detecting fluorescence.

### [Example 7] Amplification of the bands obtained by DD analysis and sequence determination thereof

A large number of arbitrary primers were used to carry out DD analysis twice. Bands that showed a different expression level between the patient and normal healthy groups were selected, and reproducible bands from two experiments were excised from the gels. Four reproducible bands, "B1001," "B1072," "B1151," and "B1466" were further analyzed.

### (a) "B1001"

Band "B1001" was found by a DD analysis using anchor primer GT15A (SEQ ID NO: 7) and arbitrary primer AG0090 (GGCTTTCGAT/SEQ ID NO: 10).

To determine the nucleotide sequence of "B1001," the gel containing the band "B1001" was excised, and DNA was eluted from the gel in a TE solution by heating at 60°C for 10 min. Using the DNA dissolved in this TE solution as a template, PCR was conducted with the same condition as the original DD-PCR, and a DNA fragment of approximately 210 bp was amplified. In this PCR, GT15A and AG0090 were used as the anchor primer and arbitrary primer, respectively. The amplified DNA fragment was cloned into plasmid vector pGEM-TEasy (Promega) to obtain plasmid p1001-01 harboring the DNA fragment of about 210 bp. The nucleotide sequence of the DNA fragment was determined using the plasmid DNA according to a conventional method.

According to a database search for homology with the gene sequences revealed that the amino acid sequence encoded by the "B1001" gene is identical to that of the SOUL gene (GenBank accession #: AF117611). However, the function of the SOUL gene hasn't been reported so far.

### (b) B1072

Band "131072" was found by a DD analysis using anchor primer GT15A (SEQ ID NO: 7) and arbitrary primer AG0096 (CAGGAGTACT/SEQ ID NO: 11).

To determine the nucleotide sequence of "B1072," the gel containing the band "B1072" was excised, and DNA was eluted from the gel in a TE solution by heating at 60°C for 10 min. Using the DNA dissolved in this TE solution as a template, PCR was conducted with the same condition as the original DD-PCR, and a DNA fragment of approximately 310 bp was amplified. In this PCR, GT15A and AG0096 were used as the anchor primer and arbitrary primer, respectively. The amplified DNA fragment was cloned into plasmid vector pGEM-TEasy (Promega) to obtain plasmid p1072-01 harboring the DNA fragment of about 310 bp. The nucleotide sequence of the DNA fragment was determined using the plasmid DNA according to conventional method. The nucleotide sequence is set forth in SEQ ID NO: 5.

According to a database search for homology with the gene sequences revealed "B1072" to be identical to the 3'-noncoding region sequence of the heat-shock protein 40 (hsp40) gene (GenBank accession# D49547). However, no function of hsp40 related to immunity or allergic disease has been reported so far.

### (c) B1151

Band "B1151" was found by a DD analysis using anchor primer GT15A (SEQ ID NO: 7) and arbitrary primer AG0103 (TGACCTGAGT/SEQ ID NO: 12).

To determine the nucleotide sequence of "B1151," the gel containing the band "B1151" was excised, and DNA was eluted from the gel in a TE solution by heating at 60°C for 10 min. Using the DNA dissolved in this TE solution as a template, PCR was conducted with the same condition as the original DD-PCR, and a DNA fragment of approximately 245 bp was amplified. In this PCR, GT15A and AG0103 were used as the anchor primer and arbitrary primer, respectively. The amplified DNA fragment was cloned into plasmid vector pGEM-TEasy (Promega) to obtain plasmid p1151-03 harboring the DNA fragment of about 245 bp. The nucleotide sequence of the DNA fragment was determined using the plasmid DNA according to conventional method. The nucleotide sequence is set forth in SEQ ID NO: 6.

According to a database search for homology with the gene sequences revealed the "B1151" to be identical with the 3'-noncoding region sequence of the vasopressin-activated calcium-mobilizing receptor like protein (VACM-1) gene (GenBank accession# X81882). However, no function of the VACM-1 gene related to immunity or allergic disease has been reported so far.

### (d) B1466

Band "131466" was found by a DD analysis using anchor primer GT15A (SEQ ID NO: 7) and arbitrary primer AG0043 (TAGTGAAGCC/SEQ ID NO: 13).

To determine the nucleotide sequence of "B1466," the gel containing the band "B1466" was excised, and DNA was eluted from the gel in a TE solution by heating at 60°C for 10 min. Using the DNA dissolved in this TE solution as a template, PCR was conducted with the same condition as the original DD-PCR, and a DNA fragment of approximately 310 bp was amplified. In this PCR, GT15A and AG0043 were used as the anchor primer and arbitrary primer, respectively. The amplified DNA fragment was cloned into plasmid vector pGEM-TEasy (Promega) to obtain plasmid p1466-02 harboring the DNA fragment of about 250 bp. The nucleotide sequence of the DNA fragment was determined using the plasmid DNA according to conventional method.

According to a database search for homology with the gene sequences revealed the "B1466" to be identical with the CDS+3'-UTR region sequence of the BRI gene (Ruben, V. et al., Nature 399 : 776-781, 1999; GenBank accession# AF152462). Furthermore, the "B1466" was identical to the sequence disclosed in international patent applications (W098/33913, W098/42783).

However, these international patent applications (W098/33913, W098/42783) describing a sequence identical to B1466, fail to disclose actual data indicating the relation of the gene to immunity or allergic disease. Likewise, the realtion of the BRI gene to immunity or allergic disease hasn't been reported so far.

### [Example 8] Quantification of expression level using ABI-7700

A part of the previously prepared whole RNA samples derived from T-cells were used for the quantification of gene expression levels by TaqMan method using ABI-PRISM 7700. This TaqMan method enables quantification of PCR-amplified DNA strands in real-time using fluorescence dyes. No quantification was carried out with whole RNA samples which was used up in the DD examination.

Primer sets prepared based on the nucleotide sequences of the DD bands determined in Example 7, or primer sets prepared based on elongation sequences that were determined in Example 10 and Example 11 based on the DD band nucleotide sequences were used.

TaqMan probe 1466 was labeled with fluorescer FAM (6-carboxyfluorescein) and TAMRA (6-carboxy-tetramethyl-rhodamine) at the 5'-end and 3'-end, respectively. Nucleotide sequences of the used primer sets and TaqMan probes are shown below.

### (a) B1001

1001F: TCGAAAGACCGTACAAACACAGTC (SEQ ID NO: 14)
1001R: GATCCACCCAGGCCTTTAGAG (SEQ ID NO: 15)
TaqMan probe 1001: TTCGGCTCTATCTTCAATGAAGACATCTG (SEQ ID NO: 16)

### (b) B1072

1072F: GCGGCTGTACCAAGAAGATGA (SEQ ID NO: 17)
1072R: CGGGCCAAAGTGGACAAG (SEQ ID NO: 18)
TaqMan probe 1072: TCCCACAAGCGGCTAAACCCCG (SEQ ID NO: 19)

### (c) B1151

1151F: ATGCAGTCTGTCTTTGGGATGAT (SEQ ID NO: 20)
1151R: CCTGTGCCTGCTTAATAAACTCAAG (SEQ ID NO: 21)
TaqMan probe 1151: AGGCCCAGCAAAAATTCATCAGGCTTT (SEQ ID NO: 22)

### (d) B1466

1466F: CGTCATCTGGTTGAAGTGCAA (SEQ ID NO: 23)
1446R: TGCATGTGCTTTGGACTAGCA (SEQ ID NO: 24)
TaqMan probe 1466: TGCTCCTCCTAGAATAACACCTGCAAGCA (SEQ ID NO: 25)

cDNAs that were used as templates were prepared by reverse transcription from total RNAs using poly-T (12 to 18 mer) as primers. To make a standard curve for the calculation of copy numbers, a plasmid clone containing the nucleotide sequence that is amplified by the respective primer set was prepared for each gene, and serial dilutions thereof were utilized as templates for the reaction. The reaction mixture composition for monitoring PCR amplification is shown in Table 3.

**Table 3**

| Reaction mixture composition for ABI-PRISM 7700 (amount per well) | |
|---|---|
| Sterile distilled water | 25.66 (µl) |
| 10x TaqMan buffer A | 5 |
| 25 mM MgCl₂ | 7 |
| dATP (10 mM) | 1.2 |
| dCTP (10 mM) | 1.2 |
| dGTP (10 mM) | 1.2 |
| dUTP (10 mM) | 1.2 |
| Forward Primer (100 µM) | 0.15 |
| Reverse Primer (100 µM) | 0.15 |
| TaqMan Probe (6.7 µM) | 1.49 |
| AmpliTaq Gold (5 U/µl) | 0.25 |
| AmpErase UNG (1 U/µl) | 0.5 |
| Template solution | 5 |
| Total volume | 50 |

In order to correct the difference of cDNA concentrations between the samples, the same quantitative analysis was carried out for β-actin gene, and its copy number was used to correct the copy number of the target genes.

Using the obtained data (Table 1), statistical analysis of significant variance in the gene expression level among each group was conducted by the non-parametric technique. The Wilcoxon test was used to compare two groups, i.e., normal healthy group (including normal healthy subjects and patients of a very mild case of allergy) and patient group (including asthma patients and atopic dermatitis patients. Subjects were also divided into three groups, the normal healthy group, asthma patient group and atopic dermatitis patient group; and the comparison between two groups among them was conducted by the Tukey's multiple comparison test. The tests were carried out using SAS Preclinical Package Version 4.0 of The SAS SYSTEM (SAS Institute, Inc.). Variance P values of the respective target genes in the Wilcoxon test and Tukey's multiple comparison test are shown in Table 4.

**Table 4**

| Test | Group comparison | B1072 | B1151 | B1001 | B1466 |
|---|---|---|---|---|---|
| Wilcoxon | normal healthy group : patient group | 0.0446 | 0.0014 | 0.0205 | 0.0112 |
| Tukey's multiple comparison | normal healthy group : atopic dermatitis group | 0.031 | 0.0005 | - | - |
| | asthma group : atopic dermatitis group | - | 0.032 | - | - |

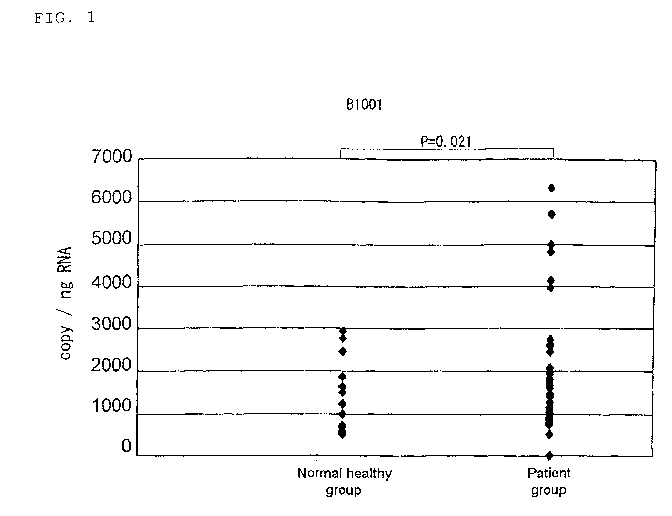

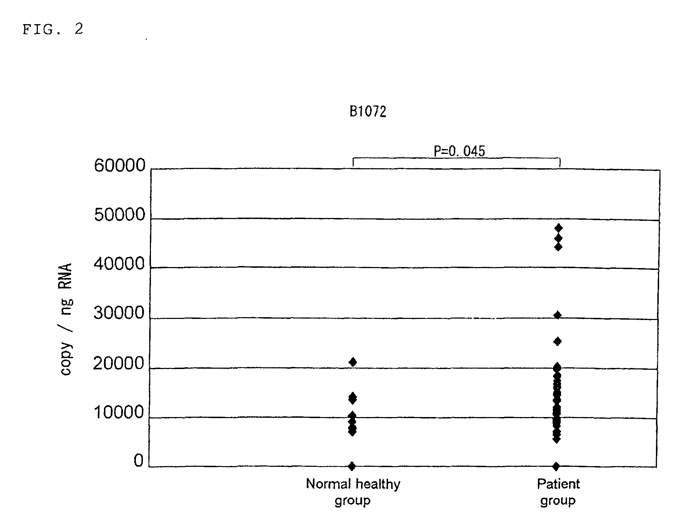

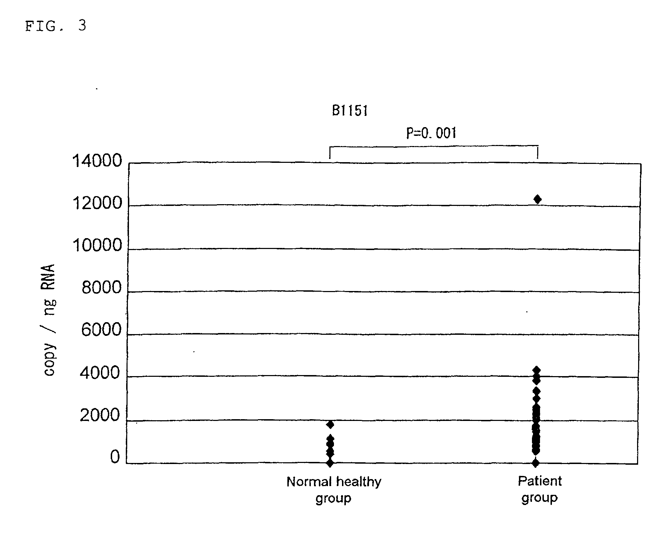

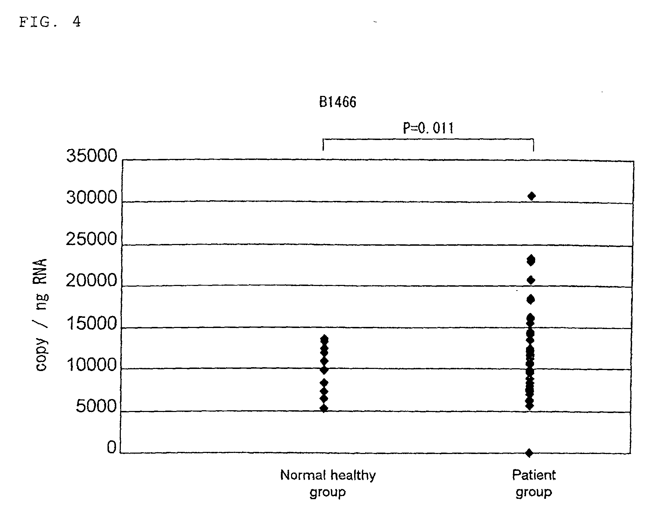

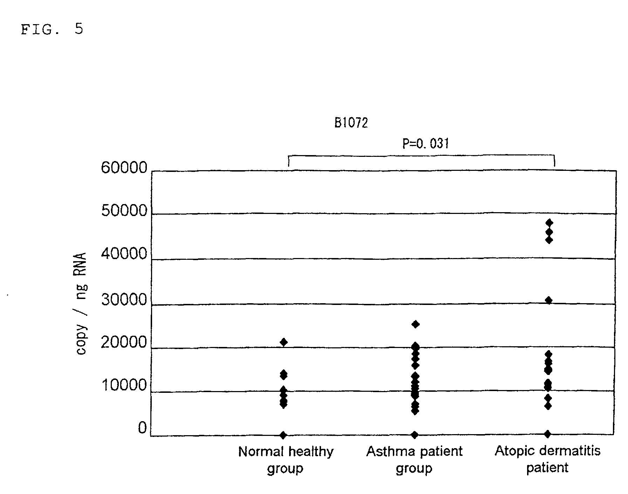

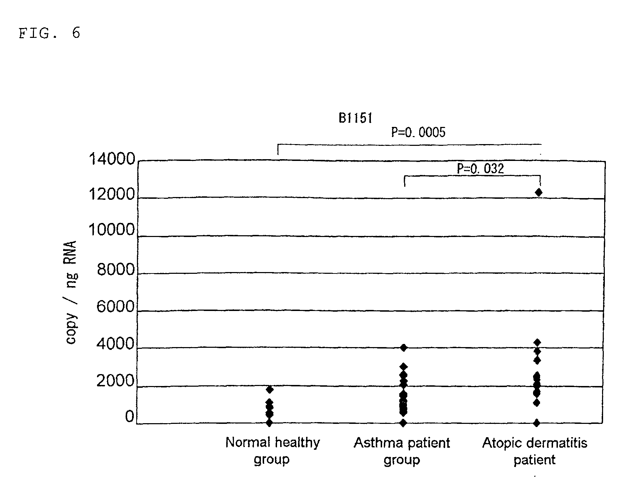

Distributions of expression levels of "B1001," "B1072," "B1151" and "B1466" wherein the subjects are divided into the normal healthy group and patient group (including both asthma patients and atopic dermatitis patients) are shown in figures 1, 2, 3, and 4, respectively. Distributions of expression levels of "B1072" and "B1151" wherein the subjects are divided into three groups (normal healthy group, asthma patient group, and atopic dermatitis patient group) are shown in figures 5 and 6, respectively. Analysis results of each gene are described below.

### (a) B1001

Expression level of each group is separately shown in Table 5. High level expression was observed in subjects of the patient group. A significant difference in the expression level of "B1001" was observed between the normal healthy group and patient group. Specifically, "B1001" expression was significantly higher in the patient group than the normal healthy group (p = 0.020). The median and mean (±SD) of the expression level (copy/ng RNA) in the normal healthy group were 1364 and 1490±866, respectively; and 1651 and 1988±1382, respectively, in the patient group.

### (b) B1072

Expression level of each group is separately shown in Table 5. High level expression was observed in the patient group, particularly in patients with atopic dermatitis. A significant difference in the expression level of "B1072" was found between the normal healthy group and patient group. Specifically, "B1072" expression was significantly higher in the patient group than in the normal healthy group (p = 0.044). The median and mean (±SD) of the expression level (copy/ng RNA) in the normal healthy group were 8545 and 10580±4468, respectively; and 12140 and 15772±10185, respectively, in the patient group.

The patient group was divided into asthma group and atopic dermatitis group. As a result of Tukey's multiple comparison test on three groups comprising the normal healthy group as well as the patient groups, the expression of "B1072" was revealed to be significantly higher in the atopic dermatitis group than the normal healthy group (p = 0.031).

### (c) B1151

Expression level of each group is separately shown in Table 5. High level expression of the B1151 gene was observed in the patient group, particularly in patients with atopic dermatitis. A significant difference in the expression level of "B1151" was found between the normal healthy group and patient group. Specifically, "B1151" expression was significantly higher in the patient group than in the normal healthy group (p = 0.0014). The median and mean (±SD) of the expression level (copy/ng RNA) in the normal healthy group were 857 and 930±421, respectively; and 1838 and 2187±1908, respectively, in the patient group.

According to the result of the Tukey's multiple comparison test, the expression level of "B1151" was revealed to be significantly higher in the atopic dermatitis patient group than in the normal healthy group (p = 0.0005); and significantly higher in the atopic dermatitis patient group than the asthma patient group (p = 0.032).

### (d) B1466

Expression level of each group is separately shown in Table 5. Patients with increased expression level of the B1466 gene could be observed in the patient group. A significant difference in the expression level of "B1466" was found between the normal healthy group and patient group. Specifically, "B1466" expression was significantly higher in the patient group than in the normal healthy group (p = 0.011). The median and mean (±SD) of the expression level (copy/ng RNA) in the normal healthy group were 10355 and 9962±3101, respectively; and 11716 and 12414+5335, respectively, in the patient group.

**Table 5**

| Gene | Function | Normal group | Patient group | Asthma patient group | Atopic dermatitis patient group |
|---|---|---|---|---|---|
| B1072 | Mean | 10580 | 15772 | 12705 | 19001 |
| | Standard deviation | 4468 | 10185 | 5195 | 13002 |
| | Median | 8545 | 12140 | 10913 | 14593 |
| B1151 | Mean | 930 | 2187 | 1612 | 2761 |
| | Standard deviation | 421 | 1908 | 909 | 2441 |
| | Median | 857 | 1838 | 1454 | 2003 |
| B1001 | Mean | 1490 | 1988 | 1773 | 2183 |
| | Standard deviation | 866 | 1382 | 1047 | 1628 |
| | Median | 1364 | 1651 | 1551 | 1673 |
| B1466 | Mean | 9962 | 12414 | 11673 | 13089 |
| | Standard deviation | 3101 | 5335 | 4235 | 6191 |
| | Median | 10355 | 11716 | 11832 | 11716 |

### [Example 9] Measurement of mRNA length of "B1001" and "B1466" by Northern hybridization

For the measurement, Human Immune System MTN Blot II and Human Cancer Cell Line MTN blot (Clontech) were employed, which are membranes transferred with mRNAs prepared from various immune-associated tissues and cancer cell lines, respectively. Using primer sets corresponding to the nucleotide sequence of each gene, PCR amplification was conducted with peripheral blood cDNA library as a template, and the obtained amplified DNA fragments were purified with PCR Product Purification Kit (QIAGEN). The purified DNA fragments were labeled with ³²P using Random Primer Labeling Kit (TAKARA) to use them as probes. Using Express Hybridization Solution (CLONTECH), Northern hybridization and membrane washing were carried out according to the attached manual. The washed membrane was exposed to an imaging plate to obtain an image using Molecular Imager System (BIO-RAD). Measurement results of respective genes are demonstrated below.

### (a) B1001

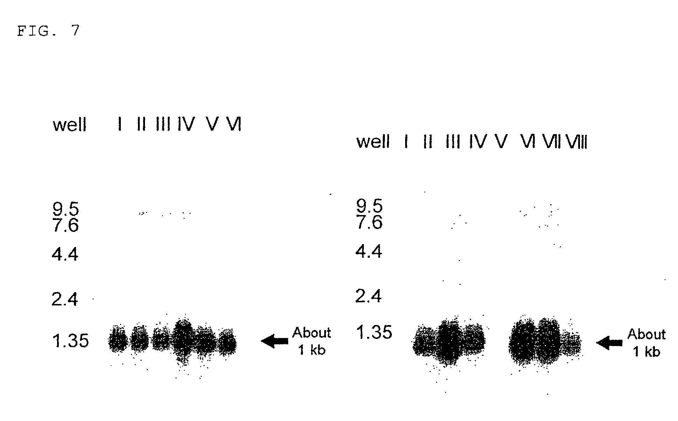

The gel electrophoretogram is shown in Fig. 7. Expression of mRNA of approximately 1.0 kb was observed in the tissues and cell lines used in the measurement.

### (b) B1466

The gel electrophoretogram is shown in Fig. 8. Expressions of mRNAs of approximately 2.1 kb and 1.3 kb were observed in the tissues and cell lines used for the measurement. Strong expression of B1466 gene was observed in the immune-associated tissues, particularly in tissues such as spleen, lymph node, and peripheral blood leukocytes, and in cancer cell lines, such as HeLa cell S3, lymphoblastic leukemia MOLT-4, colorectal adenocarcinoma SW480, and lung carcinoma A549. Thus, these results indicated that "B1466" has a function associated with immunity.

### [Example 10] Cloning and nucleotide sequence analysis of "B1466"

Using AutoAssembler (PE Applied Biosystems), ESTs that were determined to be homologous to "B1466" were clustered (assembled) to obtain sequence A of 1667 bp (SEQ ID NO: 26). Primers were designed based on this sequence A, and PCR was conducted using peripheral blood cDNA library as a template which resulted in the amplification of a PCR product of the predetermined size. Furthermore, this PCR product was sequenced using ABI377 (PE Applied Biosystems) to reveal the sequence to be identical to that of sequence A.

Then, using a pair of primers designed to correspond to a sequence within the sequence of sequence A (i.e., primers 1466-009 (5'-GCCAAGAAGGACGAGCCCAAGAG-3': SEQ ID NO: 27) and 1466-006 (5'-AAAGGGTGGGGTTATGCTGTGAT-3': SEQ ID NO: 28)) PCR was conducted to identify the target cDNA clone from Rapid-Screen cDNA Library Panel (LPS-1001: human Spleen/OriGene Technologies, Inc.). The obtained clone harbored a sequence of 1647 bp that extended about 200 bp upstream of sequence A (sequence B, SEQ ID NO: 29). From sequence A and sequence B, the sequence of "B1466" of 1868 bp was obtained. The determined sequence of the "B1466" is set forth as sequence C (SEQ ID NO: 3), and the amino acid sequence (266 a. a.) of a protein encoded by sequence C is shown as sequence D (SEQ ID NO: 4).

### [Example 11] Cloning and nucleotide sequence analysis of "B1001"

Using the information of ESTs and genomic sequence that were determined to be homologous to "B1001" (GenBank accession#: AL031003), sequence E comprising 1015 bp (SEQ ID NO: 1) was obtained. Primer 1001-006 (5'-ATGGCCGAGCCGCTCCAGCCAGAC-3', SEQ ID NO: 30) and primer 1001-007 (5'-TGTTTACAGACTACTGTCCTCTGT-3', SEQ ID NO: 31) were designed based on this sequence. Using this set of primers, PCR was conducted using peripheral blood T-cell cDNA library as a template which resulted in the amplification of a PCR product of the desired size. Furthermore, using ABI377 (PE Applide Biosystems), this PCR product was sequenced to reveal the PCR product to have an identical sequence with that of sequence E. Additionally, the amino acid sequence (205 a.a.) encoded by sequence E is shown as sequence F (SEQ ID NO: 2)

As described in the examples herein, for the first time, four genes of the present invention, "B1001," "B1072," "B1151," and "B1466", were demonstrated to have functions associated with immunity or allergic disease. These genes are extremely useful for testing for allergic diseases and screening for compounds serving as candidate therapeutic agents for allergic diseases using the expression level thereof as an index in the assay.

### Industrial Applicability

The present invention provides genes with different expression levels between normal healthy subjects and allergic disease patients. Using the expression of the genes of the present invention as an index, allergic diseases can be tested and compounds serving as therapeutic agent candidates can be screened for the disease.

Expression levels of allergic disease-associated genes provided by the present invention can be easily detected regardless of the types of allergens. Therefore, pathological conditions of allergic diseases can be comprehensively understood.

In addition, using peripheral blood leukocytes as a specimen, the expression level of genes can be analyzed in a much less invasive manner to patients according to the method for testing for allergic disease of the present invention. Furthermore, according to the gene expression analysis method of the present invention, in contrast to protein measurements such as ECP, highly sensitive measurement with a trace sample can be accomplished. Gene analysis technique trends toward high-throughput and lower prices. Therefore, the test method according to the present invention is expected to become an important bedside diagnostic method in the near future. In this sense, these genes associated with pathological conditions are highly valuable in diagnosis.

Furthermore, the screening methods of the present invention are performed using, as an index, the genes whose expression are commonly observed among allergic disease patients. Therefore, compounds that can be detected according to these screening methods are expected to be useful in controlling a wide range of allergic pathological conditions.

## Claims

1. A method of testing for allergic disease, wherein the method comprises the steps of:
a) measuring the expression level of a gene comprising the nucleotide sequence of any one of SEQ ID NOs: 1, 3, 5, and 6 in T-cells of a subject; and
b) comparing the expression level of said gene with that in T-cells of a normal healthy subject.

2. The method of claim 1, wherein the allergic disease is atopic dermatitis.

3. The method of claim 1, wherein the gene expression level is measured by PCR of cDNA.

4. The method of claim 1, wherein the gene expression level is measured by detecting a protein encoded by said gene.

5. A reagent for testing for allergic disease comprising a polynucleotide with a chain length of at least 15 nucleotides and that hybridizes with the nucleotide sequence of any one of SEQ ID NOs: 1, 3, 5, and 6, or a complementary sequence thereto.

6. A reagent for testing for allergic disease comprising an antibody that recognizes a polypeptide comprising the amino acid sequence of SEQ ID NO: 2 or 4.

7. A method of screening for compounds serving as candidate therapeutic agents for allergic disease, wherein the method comprises the steps of:
(1) contacting a candidate compound with a cell that expresses a polynucleotide selected from the group of:
(a) a polynucleotide comprising a coding region of the nucleotide sequence of any one of SEQ ID NOs: 1, 3, 5, and 6;
(b) a polynucleotide encoding a protein comprising the amino acid sequence of SEQ ID NO: 2 or 4;
(c) a polynucleotide encoding a protein comprising the amino acid sequence of SEQ ID NO: 2 or 4 wherein one or more amino acids are substituted, deleted, inserted and/or added, and the expression level of the protein is increased in accordance with allergic disease; and
(d) a polynucleotide hybridizing under stringent conditions to a DNA consisting of the nucleotide sequence of any one of SEQ ID NOs: 1, 3, 5, and 6, wherein the polynucleotide encodes a protein whose expression level increases in accordance with allergic disease;
(2) measuring the expression level of the polynucleotide of any one of (a) to (d); and
(3) selecting the compound that reduces the expression level of said polynucleotide compared to a control.

8. The method of claim 7, wherein the cell is a T-cell.

9. A kit for screening for compounds serving as candidate therapeutic agents for allergic disease, which comprises a polynucleotide having a chain length of at least 15 nucleotides that hybridizes to the nucleotide sequence of any one of SEQ ID NOs: 1, 3, 5, and 6 or a sequence complementary thereto, and a cell that expresses a gene comprising the nucleotide sequence of any one of SEQ ID NOs: 1, 3, 5, and 6.

10. A kit for screening for compounds serving as candidate therapeutic agents for allergic disease, which comprises an antibody that recognizes a peptide comprising the amino acid sequence of either SEQ ID NO: 2 or 4, and a cell that expresses a gene comprising the nucleotide sequence of either SEQ ID NO: 1 or 3.

11. An allergic disease animal model consisting of a non-human transgenic vertebrate with an increased expression level in T-cells of the polynucleotide selected from the group of:
(a) a polynucleotide comprising the coding region of the nucleotide sequence of any one of SEQ ID NOs: 1, 3, 5, and 6;
(b) a polynucleotide encoding a protein comprising the amino acid sequence of either SEQ ID NO: 2 or 4;
(c) a polynucleotide encoding a protein comprising the amino acid sequence of either SEQ ID NO: 2 or 4, wherein one or more amino acids are substituted, deleted, inserted and/or added, and the expression level of the protein is increased in accordance with allergic disease; and
(d) a polynucleotide hybridizing under stringent conditions to a DNA consisting of a nucleotide sequence of any one of SEQ ID NOs: 1, 3, 5, and 6 , wherein the polynucleotide encodes a protein whose expression level is increased in accordance with allergic disease.

12. A method of screening for compounds serving as candidate therapeutic agents for allergic disease, wherein the method comprises the steps of:
(1) administering a candidate compound to the animal model of claim 11;
(2) measuring the expression level of the polynucleotide of any one of (a) to (d) of claim 11 in T-cells; and
(3) selecting the compound that reduces the expression level of said polynucleotide compared to a control.

13. A method of screening for compounds serving as candidate therapeutic agents for allergic disease, wherein the method comprises the steps of:
(1) contacting a candidate compound with a cell transfected with a vector containing a transcriptional regulatory region of a gene comprising the nucleotide sequence of any one of SEQ ID NOs: 1, 3, 5, and 6, and a reporter gene that is expressed under the control of the transcriptional regulatory region;
(2) measuring the activity of said reporter gene; and
(3) selecting the compound that reduces said activity compared to a control.

14. A therapeutic agent for allergic disease containing a compound that can be obtained by the screening method of any one of claims 7, 12, and 13 as the principal ingredient.

15. A therapeutic agent for allergic disease containing an antisense DNA of the nucleotide sequence of any one of SEQ ID NOs: 1, 3, 5, and 6, or a portion thereof as the principal ingredient.

16. A therapeutic agent for allergic disease containing an antibody that recognizes a peptide comprising the amino acid sequence of either SEQ ID NO: 2 or 4 as the principal ingredient.
